# EUROPEAN PATENT APPLICATION

(11) **EP 1 818 327 A1**
(43) Date of publication of application: **15.08.2007**
(21) Application number: 05809648.8
(22) Date of filing: 28.11.2005
(51) Int. Cl.: C07D 303/32, C07D 301/06, C07D 301/12, C07D 301/19, C08F 220/26, C08F 220/10, C08F 212/08, C08F 222/40, C07B 61/00

(54) **ALICYCLIC EPOXY (METH)ACRYLATES, PROCESS FOR PRODUCTION THEREOF, AND COPOLYMERS**

(30) Priority: 30.11.2004 JP 2004346860; 14.12.2004 JP 2004361381
(71) Applicant: DAICEL CHEMICAL INDUSTRIES, LTD., Sakai-shi, Osaka 590-8501 (JP)
(72) Inventor: TAKAI, Hideyuki, Otake-shi Hiroshima 739-0651 (JP); IYOSHI, Shuso, Himeji-shi Hyogo 671-1204 (JP); NIJUKKEN, Toshihiko, Kobe-shi Hyogo 655-0018 (JP); MORI, Misao, Matsudo-shi Chiba 270-0035 (JP)
(74) Representative: Grünecker, Kinkeldey, Stockmair & Schwanhäusser Anwaltssozietät
(86) International application number: PCT/JP2005/021794
(87) International publication number: WO 2006/059564

(57) **Abstract**

There are provided a novel compound having an 3,4-epoxytricyclo[5.2.1.0^{2,6}]decane skeleton and a polymerizable unsaturated bond, and a process for the preparation of the compound.

There is also provided a copolymer including a monomeric unit (A) containing an alkali-soluble group, and a monomeric unit (B) corresponding to an epoxy-containing polymerizable unsaturated compound, or a copolymer including, in addition to the monomeric units (A) and (B), a monomeric unit (C) corresponding to an epoxy-free polymerizable unsaturated compound such as a N-substituted maleimide, in which the monomeric unit (B) occupies 40 to 90 percent by weight of total monomeric units, and 30 percent by weight or more of the monomeric unit (B) is a monomeric unit corresponding to a specific 3,4-epoxytricyclo[5.2.1.0^{2,6}]decane skeleton-containing compound. The copolymer can yield a film excellent typically in thermal stability, yield a resin composition having very high storage stability, and be easily synthetically prepared.

## Description

### Technical Field

The present invention relates to cycloaliphatic epoxy unsaturated carboxylic acid esters structurally containing epoxy group (unsaturated carboxylic acid esters having an 3,4-epoxytricyclo[5.2.1.0^{2,6}]decane skeleton), such as cycloaliphatic epoxy (meth)acrylates, and preparation processes thereof. These cycloaliphatic epoxy (meth)acrylates, other esters, and resins obtained therefrom are useful as materials for coating agents, inks, adhesives, sealants, resist materials, and optically transparent sheets. The present invention also relates to copolymers containing a structural unit having an 3,4-epoxytricyclo[5.2.1.0^{2,6}]decane skeleton, and preparation processes thereof. More specifically, it relates to copolymers containing a structural unit having an 3,4-epoxytricyclo[5.2.1.0^{2,6}]decane ring, and preparation processes thereof. These copolymers are suitable as radiation-sensitive resins for use in lithography techniques in semiconductor processes using active rays such as far-ultraviolet rays, electron beams, ion beams, and X-rays; and for use as materials for the formation of dielectric films and protecting films arranged in electronic parts such as liquid crystal display devices, integrated circuit devices, and solid-state imaging devices.

### Background Art

Alicyclic (meth)acrylates and (meth)acrylates having a cycloaliphatic epoxy group (unsaturated carboxylic acid ester compounds having a cycloaliphatic epoxy group) are useful in the fields typically of coating, inks, adhesives, and sealants. Among them, polymers and cured articles obtained from (meth)acrylates containing both an alicyclic structure and an epoxy group are excellent in weather resistance and have properties suitable in outdoor use. This is because epoxy group contained in the alicyclic structure is reactive in a ring-opening reaction, and a radical polymerizable double bond in the (meth)acrylate structure is reactive, and these moieties allow the polymers and cured articles to exhibit such performance.

Examples of known unsaturated carboxylic acid esters intramolecularly having an epoxy group include terminal epoxy-containing (meth)acrylates such as glycidyl methacrylate and 1-methyl-1,2-epoxy-ethyl methacrylate; and (meth)acrylates having a cycloaliphatic epoxy group, such as 3,4-epoxycyclohexylmethyl acrylate (a product of Daicel Chemical Industries, Ltd. under the trade name of "CYCLOMER A-200") and 3,4-epoxycyclohexylmethyl methacrylate (a product of Daicel Chemical Industries, Ltd. under the trade name of "CYCLOMER M-100"). U.S. Pat. No. 3,536,687 discloses an ester compound of an α,β-unsaturated acid with a cyclohexenyl-alkyl alcohol and an alkylcyclohexenyl-alkyl alcohol; and a homopolymer and a copolymer containing the ester compound as a monomeric component.

In addition, "Journal of the American Chemical Society" vol. 81, p. 3350 (1959) describes 3,4-epoxycyclohexylmethyl acrylate and 3,4-epoxy-6-methylcyclohexylmethyl acrylate. Deutsches Reichspatent (German Patent) No. 1063808 (August 20, 1959) ("Chemical Abstracts" 55:14983e) discloses methacrylate of 3,4-epoxycyclohexylmethanol which may be substituted with an alkyl group having twelve or less carbon atoms and, in particular, which may be substituted with methyl group on the carbon at the 6-position. These compounds are subjected to polymerization alone or in combination with another olefinic compound, and produced polymers are cured as a result of a reaction of epoxy group.

Japanese Unexamined Patent Application Publication (JP-A) No. 02-18410 discloses the preparation of a coating for an erasable white board by curing siloxane methacrylate with 3,4-epoxycyclohexylmethyl methacrylate. Japanese Unexamined Patent Application Publication (JP-A) No. 57-47365 mentions that a copolymer containing acrylic acid or 3,4-epoxycyclohexylmethyl (meth)acrylate as a structural unit is effective in a thermosetting powdery coating composition.

Such cycloaliphatic epoxy (meth)acrylates typified by above-mentioned CYCLOMERs are used as materials typically for polymers. These compounds, however, are insufficient in storage stability when epoxy-containing acrylic polymers are prepared as a result of addition polymerization of cycloaliphatic epoxy (meth)acrylates, and the epoxy-containing acrylic polymers are stored together with an acid group-containing curing agent. This is because the epoxy group in these compounds are highly reactive with an acid, as with glycidyl epoxy group.

Demands have been increasingly made to provide devices with larger packing densities and higher accumulation in the production of electronic devices which require micromachining on the order of submicrons, such as in very-large-scale integrated circuits (VLSIs). To meet these demands, more and more requirements have been made on photolithography techniques for fine patterning. Electronic parts such as liquid crystal display devices, integrated circuit devices, and solid-state imaging devices may include, for example, protective films for preventing deterioration and damage of the electronic parts; interlayer dielectric films arranged for insulating between layered interconnections; planarizing films for planarizing the surface of devices; and dielectric films for maintaining electric insulation. Of these devices, liquid crystal display devices are prepared, for example, in the following manner. A TFT-type thin-film transistor (THT) is formed and covered with an interlayer dielectric film to form a back substrate. Separately, a polarizer is formed on a glass plate, a black matrix layer and a color filter layer are patterned according to necessity, and a transparent electroconductive circuit layer and an interlayer dielectric film are sequentially formed to yield a front substrate. The front substrate and the back substrate are arranged so as to face each other with the interposition of a spacer, and a liquid crystal is sealed in between the two substrates to complete a liquid crystal display device. Photosensitive resin compositions for use therein are required to provide excellent transparency, light resistance, developability, and surface smoothness.

Chemically amplified resists using light-activatable acid generators as photosensitizers are well known as resists having higher sensitivity. Using such a chemically amplified resist, higher sensitivity is available, for example, in the following manner. A resin composition containing a light-activatable acid generator and a resin having an epoxy-containing structural unit is exposed to light to allow the light-activatable acid generator to generate a protic acid, and the protic acid acts to cleave the epoxy group to cause a crosslinking reaction. This makes the resin insoluble in a developer to thereby form a pattern. In addition, heat treatment is conducted after light exposure, this allows the resist to move in a resist solid phase, and the acid acts to catalytically amplify chemical changes of the resist resin and other components. Thus, dramatically high sensitivity is achieved, as compared with that in a regular resist having a photoreaction efficiency (reaction per one photon) of less than 1. The great majority of currently developed resists are chemically amplified resists. Irradiation light sources have shorter and shorter wavelengths, and materials usable therein should have higher and higher sensitivities. To develop such high-sensitivity materials, the chemical amplification mechanism should essentially be employed.

On the other hand, radiation-sensitive resin compositions are generally used as materials for forming dielectric films in THT liquid crystal display devices and integrated circuit devices, because these dielectric film should be subjected to fine machining. Such radiation-sensitive resin compositions are required to have high radiation sensitivity. The dielectric films are also required to have excellent solvent resistance. This is because, if dielectric films have poor solvent resistance, the dielectric films may undergo swelling, deformation, or delamination from substrates due to organic solvents, and this may cause serious problems in the production of liquid crystal display devices and integrated circuit devices. In addition, dielectric films, if used in liquid crystal display devices and solid-state imaging devices, should have high optical transparency according to necessity.

In order to meet these requirements, Japanese Unexamined Patent Application Publication (JP-A) No. 2003-7612 discloses a photosensitive resin composition containing a copolymer between a cycloaliphatic epoxy-containing polymerizable unsaturated compound and a radical polymerizable compound, in which an unsaturated carboxylic acid, for example, is used as the radical polymerizable compound. Such epoxy compounds are effective for yielding films having good etching resistance in optically amplified resists, because they are readily crosslinked by the action of an acid generated by a light-activatable acid generator and heating thereafter (post-baking). Although being highly cationically polymerizable, the cycloaliphatic epoxy-containing polymerizable unsaturated compound is susceptible to a reaction with a carboxyl group derived from an unsaturated carboxylic acid used for providing alkali solubility, is thereby poor in storage stability, and should be stored at low temperatures of -20°C or lower. This substantially prevents the practical use of the compound.

Japanese Patent No. 305549 discloses a photosensitive resin composition including a copolymer containing a monomeric unit corresponding to a (meth)acrylic acid ester having an oxygen atom in ester moiety directly bound to an organic cyclic hydrocarbon group; a monomeric unit having an epoxy-containing hydrocarbon group; and a carboxyl-containing monomeric unit. Such (meth)acrylic acid esters having an oxygen atom in ester moiety directly bound to an organic cyclic hydrocarbon group, however, are often difficult to prepare as monomers, because they have a very bulky group at the adjacent position to the ester group. In addition, they have poor solubility in organic solvents and are difficult to handle in polymerization reactions. The (meth)acrylic acid esters having an oxygen atom in ester moiety directly bound to an organic cyclic hydrocarbon group may difficult to yield a uniform polymer and thereby fail to provide desired resist performance, because they have very low polarity and may cause uneven distribution in monomer composition of a polymer when they are subjecting to copolymerization with an unsaturated carboxylic acid or an epoxy-containing monomer having high polarity.

Patent Document 1: U.S. Pat. No. 3536687
Patent Document 2: German Patent No. 1063808
Patent Document 3: Japanese Unexamined Patent Application Publication (JP-A) No. 02-18410
Patent Document 4: Japanese Unexamined Patent Application Publication (JP-A) No. 57-47364
Patent Document 6: Japanese Unexamined Patent Application Publication (JP-A) No. 2003-7612
Patent Document 6: Japanese Patent No. 3055495
Non-patent Document 1: "Journal of the American Chemical Society" vol. 81, p. 3350 (1959)

### Disclosure of Invention

### Problems to be Solved by the Invention

An object of the present invention is to provide a novel compound having a radical polymerizable double bond and a cycloaliphatic epoxy group, a preparation process thereof, and a copolymer having a monomeric unit corresponding to the compound.

Another object of the present invention is to provide a polymer and a preparation process thereof, which polymer is, if used as a radiation-sensitive resin, highly soluble in solvents, can yield a high-performance film (coat) having excellent optical transparency, thermal stability, etching resistance, planarity, and developability, can yield a resin composition having very high storage stability, and is easily synthetically prepared.

### Means for Solving the Problems

The present inventors made intensive investigations to achieve the above objects and focused attention on unsaturated carboxylic acid esters having a dicyclopentenyl group. When a compound having a dicyclopentenyl group, such as dicyclopentenyl (meth)acrylate, is introduced from commercially available dicyclopentadiene (DCPD), a dicyclopentenyl group corresponding to dicyclopentadiene in the compound has either of structures (a) and (b) represented by following Formula (5): 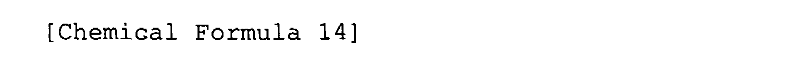 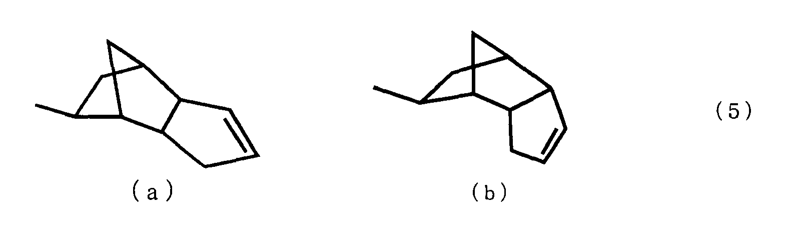 The compound includes a compound having a group represented by the structure (b) as a major product. When the double bond in the group represented by the structure (b) is epoxidized, the resulting epoxy group might have poor reactivity, because the compound may have a structure in which the epoxy group turns into the tricyclodecane ring and is hindered by the ring. Accordingly, the epoxy group in 3,4-epoxytricyclo[5.2.1.0^{2,6}]decane ring is expected to have lower reactivity than that of epoxy group in conventional cycloaliphatic epoxy compounds such as 3,4-epoxytricyclohexyl (meth)acrylates. Specifically, a polymer prepared by using a polymerizable compound containing an 3,4-epoxytricyclo[5.2.1.0^{2,6}]decane ring as a monomeric component is expected to have improved storage stability. In particular, it is expected to have improved stability as a composition further containing an acid group-containing curing agent, because the epoxy group in the polymer has suppressed reactivity for the above-mentioned reason. In addition, a polymer prepared by using a compound having a tricyclodecane ring as a monomeric component has a higher glass transition temperature (Tg) due to the bulky skeleton of the tricyclodecane ring. The polymer is therefore expected to have, for example, higher optical transparency and higher thermal stability than resins prepared typically from cycloaliphatic epoxy (meth)acrylate compounds in related art. In addition, when the polymer (resin) is formed into a coat, the stickiness of the coat can be more effectively prevented.

After making further investigations, the present inventors found that a novel compound having a radical polymerizable double bond and a cycloaliphatic epoxy group can be obtained by using specific materials and specific processes, and that, when a polymer is prepared by the copolymerization of a monomer mixture containing a specific amount of the compound having a radical polymerizable double bond and a cycloaliphatic epoxy group with a polymerizable unsaturated compound having an alkali-soluble group or a monomer mixture further containing a specific polymerizable unsaturated compound in addition to these components, the polymer is highly soluble in solvents, has very good storage stability, if stored in the form of a solution, and can yield a film (coat) excellent typically in optical transparency, thermal stability, etching resistance, planarity, and developability. The present invention has been made based on these findings.

Specifically, according to the present invention, there is provided an unsaturated carboxylic acid ester containing an 3,4-epoxytricyclo[5.2.1.0^{2,6}]decane skeleton, represented by following Formula (1a): 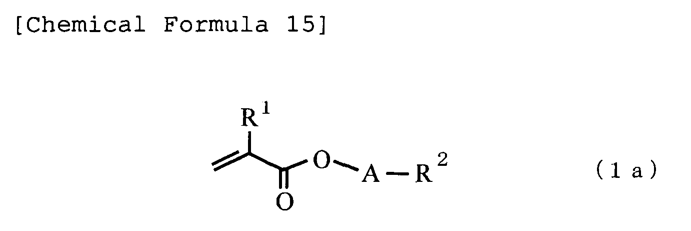 wherein R¹ represents a hydrogen atom or an alkyl group having one to seven carbon atoms which may be substituted with hydroxyl group; "A" represents a single bond or a bivalent hydrocarbon group which may contain hetero atom; and R² represents a group having an 3,4-epoxytricyclo[5.2.1.0^{2,6}]decane skeleton, represented by following Formula (1b): 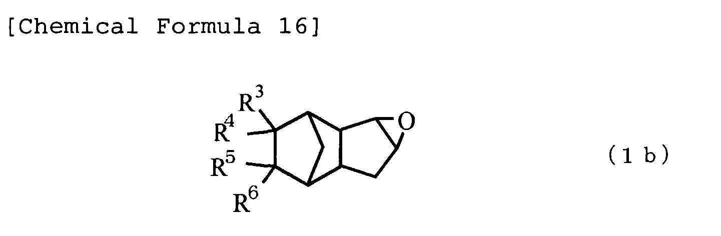 wherein one of R³, R⁴, R⁵, and R⁶ represents a bond with "A" in Formula (1a) and the others each represent a hydrogen atom or an alkyl group having one to ten carbon atoms.

"A" in Formula (1a) is preferably a group represented by following Formula (2): 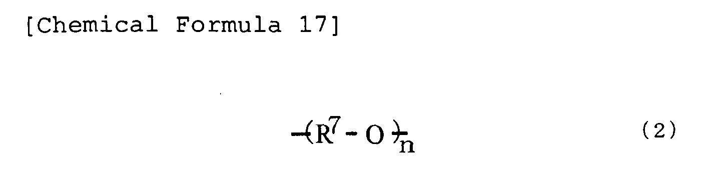 wherein R⁷ represents a bivalent hydrocarbon group having one to twelve carbon atoms; and "n" represents an integer of 0 or more.
The group R⁷ is especially preferably an ethylene group.
The repetition number "n" is preferably an integer of 0 to 5.

According to the present invention, there is also provided a process for the preparation of an unsaturated carboxylic acid ester containing an 3,4-epoxytricyclo[5.2.1.0^{2,6}]decane skeleton, including the step of carrying out epoxidation of an unsaturated carboxylic acid ester containing a tricyclo[5.2.1.0^{2,6}]dec-3-ene skeleton, represented by following Formula (3a): 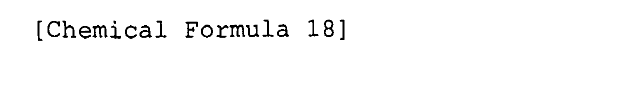 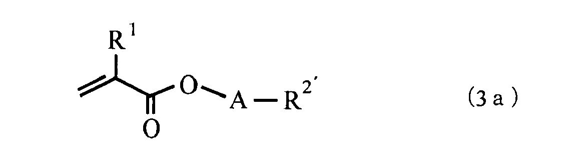 wherein R¹ represents a hydrogen atom or an alkyl group having one to seven carbon atoms which may be substituted with hydroxyl group; "A" represents a single bond or a bivalent hydrocarbon group which may contain hetero atom; and R²' represents a group having a tricyclo[5.2.1.0^{2,6}]dec-3-ene skeleton, represented by following Formula (3b): 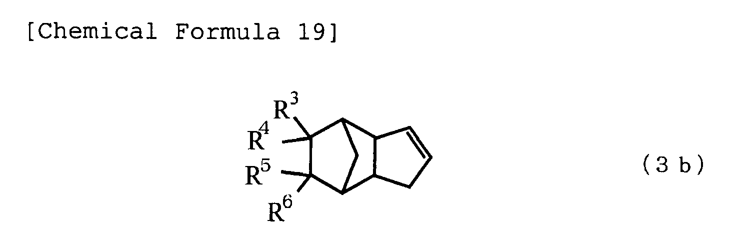 wherein one of R³, R⁴, R⁵, and R⁶ represents a bond with "A" in Formula (3a), and the others each represent a hydrogen atom or an alkyl group having one to ten carbon atoms, in the presence of an epoxidizing agent selected from the group consisting of a peracid prepared as a result of oxidation of an aldehyde; hydrogen peroxide; and a peracid derived from hydrogen peroxide and an organic acid, to thereby yield an unsaturated carboxylic acid ester containing an 3,4-epoxytricyclo[5.2.1.0^{2,6}]decane skeleton, represented by following Formula (1a): 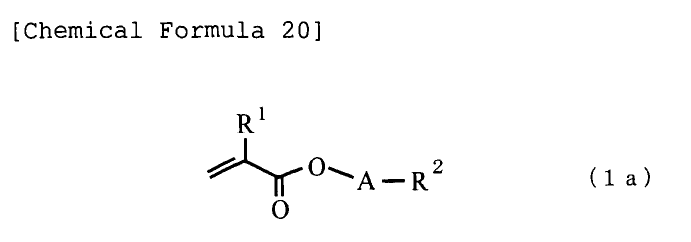 wherein R¹ represents a hydrogen atom or an alkyl group having one to seven carbon atoms which may be substituted with hydroxyl group; "A" represents a single bond or a bivalent hydrocarbon group which may contain hetero atom; and R² represents a group having an 3,4-epoxytricyclo[5.2.1.0^{2,6}]decane skeleton, represented by following Formula (1b): 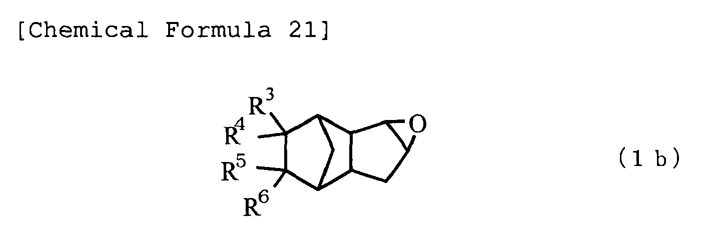 wherein one of R³, R⁴, R⁵, and R⁶ represents a bond with "A" in Formula (1a), and the others each represent a hydrogen atom or an alkyl group having one to ten carbon atoms.

Peracetic acid is preferably used as the peracid.

When hydrogen peroxide is used as the epoxidizing agent, epoxidation is preferably carried out further in the presence of a phase transfer catalyst and a metal salt of tungstic acid.

There is also provided, according to the present invention, a copolymer containing a monomeric unit corresponding to an unsaturated carboxylic acid ester containing an 3,4-epoxytricyclo[5.2.1.0^{2,6}]decane skeleton, represented by following Formula (1a): 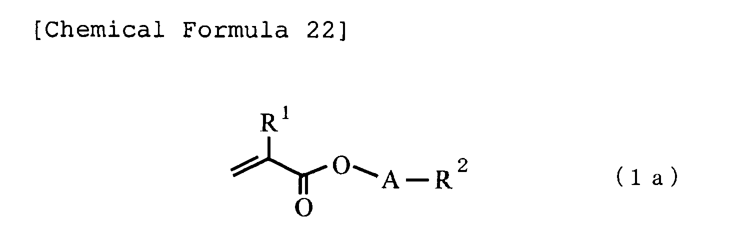 wherein R¹ represents a hydrogen atom or an alkyl group having one to seven carbon atoms which may be substituted with hydroxyl group; "A" represents a single bond or a bivalent hydrocarbon group which may contain hetero atom; and R² represents a group having an 3,4-epoxytricyclo[5.2.1.0^{2,6}]decane skeleton, represented by following Formula (1b): 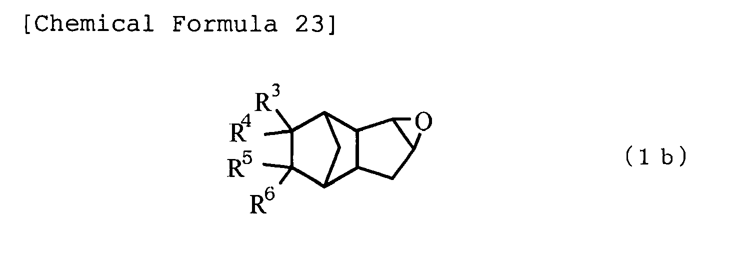 wherein one of R³, R⁴, R⁵, and R⁶ represents a bond with "A" in Formula (1a), and the others each represent a hydrogen atom or an alkyl group having one to ten carbon atoms, wherein the copolymer is a copolymer including monomeric units (A) containing an alkali-soluble group and monomeric units (B) corresponding to an epoxy-containing polymerizable unsaturated compound, or a copolymer including, in addition to the monomeric units (A) and (B), monomeric units (C) corresponding to at least one epoxy-free polymerizable unsaturated compound selected from the group consisting of (c1) an alkyl-substituted or -unsubstituted styrene, (c2) an unsaturated carboxylic acid ester represented by following Formula (4): 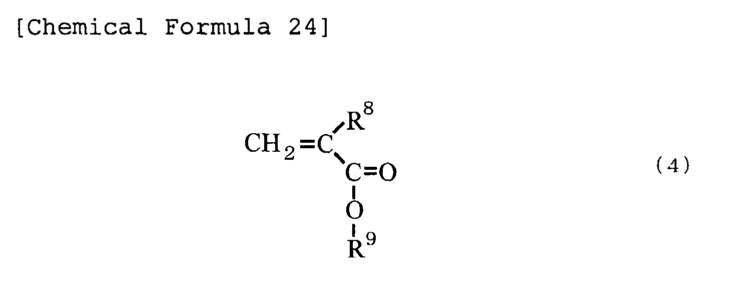 wherein R⁸ represents a hydrogen atom or an alkyl group having one to seven carbon atoms; R⁹ represents a primary or secondary alkyl group having one to twelve carbon atoms, an alkenyl group having two to twelve carbon atoms, an aryl group, an aralkyl group, a group containing a cyclic ether having four or more members, or a -(R¹⁰-O)ₘ-R¹¹ group, wherein R¹⁰ represents a bivalent hydrocarbon group having one to twelve carbon atoms; R¹¹ represents a hydrogen atom or a hydrocarbon group; and "m" represents an integer of 1 or more,
and (c3) an N-substituted maleimide, wherein the monomeric units (B) occupy 40 to 90 percent by weight of the total monomeric units, and wherein 30 percent by weight or more of the monomeric units (B) are monomeric units corresponding to the unsaturated carboxylic acid ester containing an 3,4-epoxytricyclo[5.2.1.0^{2,6}]decane skeleton, represented by Formula (1a).

In the copolymer, the monomeric units (A) preferably occupy 10 to 50 percent by weight of total monomeric units.

A copolymer according to the present invention can be advantageously used as a resin for a photoresist soluble in an aqueous alkali solution

According to the present invention, there is also provided a process for the preparation of a copolymer, including the step of subjecting a monomer mixture to copolymerization, wherein the monomer mixture is a monomer mixture containing a polymerizable unsaturated compound (a) having an alkali-soluble group, and an epoxy-containing polymerizable unsaturated compound (b), or is a monomer mixture further containing, in addition to the compounds (a) and (b), at least one epoxy-free polymerizable unsaturated compound (c) selected from the group consisting of (c1) an alkyl-substituted or -unsubstituted styrene, (c2) an unsaturated carboxylic acid ester represented by following Formula (4): 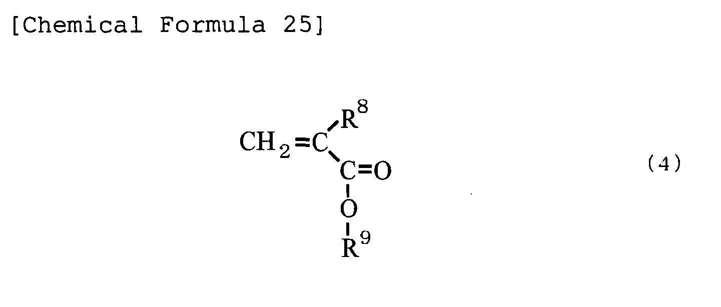 wherein R⁸ represents a hydrogen atom or an alkyl group having one to seven carbon atoms; R⁹ represents a primary or secondary alkyl group having one to twelve carbon atoms, an alkenyl group having two to twelve carbon atoms, an aryl group, an aralkyl group, a group containing a cyclic ether having four or more members, or a-(R¹⁰-O)ₘ-R¹¹ group, wherein R¹⁰ represents a bivalent hydrocarbon group having one to twelve carbon atoms; R¹¹ represents a hydrogen atom or a hydrocarbon group; and "m" represents an integer of 1 or more, and
(c3) an N-substituted maleimide, wherein the epoxy-containing polymerizable unsaturated compound (b) occupies 40 to 90 percent by weight of total monomers, and wherein 30 percent by weight or more of the epoxy-containing polymerizable unsaturated compound (b) is an unsaturated carboxylic acid ester containing an 3,4-epoxytricyclo[5.2.1.0^{2,6}]decane skeleton, represented by following Formula (1a): 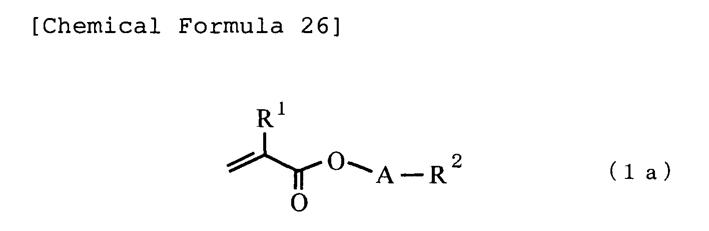 wherein R¹ represents a hydrogen atom or an alkyl group having one to seven carbon atoms which may be substituted with hydroxyl group; "A" represents a single bond or a bivalent hydrocarbon group which may contain hetero atom; and R² represents a group having an 3,4-epoxytricyclo[5.2.1.0^{2,6}]decane skeleton, represented by following Formula (1b): 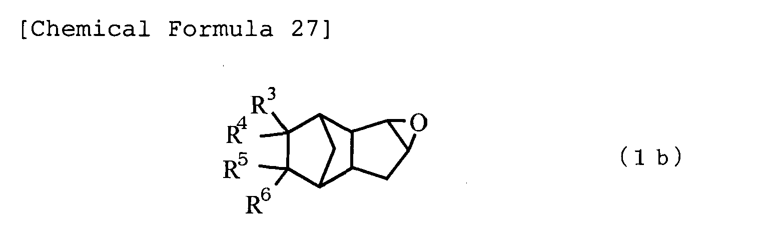 wherein one of R³, R⁴, R⁵, and R⁶ represents a bond with "A" in Formula (1a), and the others each represent a hydrogen atom or an alkyl group having one to ten carbon atoms. Advantages

According to the present invention, there is provided a novel unsaturated carboxylic acid ester compound having a 3,4-epoxytricyclo[5.2.1.0^{2,6}]decane skeleton (a cycloaliphatic epoxy (meth)acrylate compound having a radical polymerizable double bond). The unsaturated carboxylic acid ester includes an epoxy group having suppressed reactivity and is excellent in storage stability when stored together with an acid group-containing curing agent.

A copolymer according to the present invention is highly soluble in solvents, is highly stably stored, and can yield a film (coat) which is excellent typically in optical transparency, such as optical transparency to light having wavelengths of 180 to 400 nm, thermal stability, etching resistance, planarity, and developability. The copolymer is therefore very useful as a resin for a photoresist.

### Brief Description of the Drawings

FIG. 1 is a ¹H-NMR chart of an unsaturated carboxylic acid ester containing a tricyclo[5.2.1.0^{2,6}]dec-3-ene skeleton (alicyclic acrylate) (a product of Hitachi Chemical Co., Ltd. under the trade name of "FA-511A") used in Example 1.
FIG. 2 is a ¹H-NMR chart of an unsaturated carboxylic acid ester containing an 3,4-epoxytricyclo [5.2.1.0^{2,6}]decane skeleton (cycloaliphatic epoxy acrylate) prepared in Example 1.

### Best Mode for Carrying Out the Invention

Unsaturated carboxylic acid esters containing an 3,4-epoxytricyclo[5.2.1.0^{2,6}]decane skeleton (cycloaliphatic epoxy (meth)acrylates, and other cycloaliphatic epoxy unsaturated carboxylic acid esters) according to the present invention will be illustrated in detail below. In Formula (1a), R¹ represents a hydrogen atom or an alkyl group having one to seven carbon atoms which may be substituted with hydroxyl group; "A" represents a single bond or a bivalent hydrocarbon group which may contain hetero atom; and R² represents a group having an 3,4-epoxytricyclo[5.2.1.0^{2,6}]decane skeleton, represented by Formula (1b).

Examples of the alkyl group having one to seven carbon atoms which may be substituted with hydroxyl group as R¹ include alkyl groups such as methyl, ethyl, propyl, isopropyl, butyl, isobutyl, s-butyl, pentyl, hexyl, and heptyl groups; and hydroxyalkyl groups such as hydroxymethyl group, 1-hydroxyethyl group, 2-hydroxyethyl group, 1-hydroxypropyl group, 2-hydroxypropyl group, 3-hydroxypropyl group, 1-hydroxy-1-methylethyl group, 2-hydroxy-1-methylethyl group, 1-hydroxybutyl group, 2-hydroxybutyl group, 3-hydroxybutyl group, and 4-hydroxybutyl group. R¹ is preferably a hydrogen atom or an alkyl group having one or two carbon atoms which may be substituted with hydroxyl group, and is especially preferably a hydrogen atom or a methyl group.

Examples of the bivalent hydrocarbon group which may contain hetero atom as "A" include bivalent hydrocarbon groups; and bivalent groups each containing one or more bivalent hydrocarbon groups and one or more hetero atoms. In bivalent hydrocarbon groups each containing one or more hetero atoms, the hetero atom may be bound at the terminal of a hydrocarbon group or may be interposed between carbon atoms constituting the hydrocarbon group. Such hetero atoms include nitrogen, oxygen, and sulfur atoms.

A representative example of "A" includes a group represented by following Formula (2): 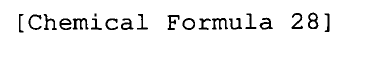 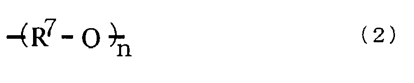 wherein R⁷ represents a bivalent hydrocarbon group having one to twelve carbon atoms; and "n" represents an integer of 0 or more.

Examples of the bivalent hydrocarbon group having one to twelve carbon atoms as R⁷ include bivalent straight- or branched-chain alkylene groups such as methylene, ethylene, propylene, trimethylene, tetramethylene, hexamethylene, octamethylene, decamethylene, and dodecamethylene groups; bivalent alicyclic hydrocarbon groups including cycloalkylene groups, cycloalkylidene groups, and bivalent bridged carbocyclic groups, such as cyclopentylene, cyclohexylene, cyclopentylidene, and cyclohexylidene; and bivalent hydrocarbon groups including two or more of these groups combined with each other. Preferred examples of R⁷ include alkylene groups having one to six carbon atoms, such as methylene, ethylene, propylene, tetramethylene, and hexamethylene groups; and alicyclic hydrocarbon groups having three to six members, such as cyclohexylene group. The repetition number "n" is preferably an integer of 0 to 10, more preferably an integer of 0 to 5, further preferably an integer of 0 to 4, and especially preferably 0 or 1.

Other representative examples of "A" include alkylene groups including alkylene groups having one to twelve carbon atoms, such as methylene group, ethylene group, propylene group, and trimethylene group, of which alkylene groups having one to six carbon atoms are preferred; thioalkylene groups including thioalkylene groups having one to twelve carbon atoms, such as thiomethylene group, thioethylene group, and thiopropylene group, of which thioalkylene groups having one to six carbon atoms are preferred; and aminoalkylene groups including aminoalkylene groups having one to twelve carbon atoms, such as aminomethylene group, aminoethylene group, and aminopropylene group, of which aminoalkylene groups having one to six carbon atoms are preferred.

"A" is preferably a single bond [a group of Formula (2) wherein "n" is 0 ], an alkylene group having one to six carbon atoms (of which an alkylene group having one to three carbon atoms is more preferred), or an oxyalkylene group having one to six carbon atoms, of which an oxyalkylene group having two or three carbon atoms is more preferred [a group of Formula (2) wherein "n" is 1; and R⁷ is an alkylene group having one to six carbon atoms, of which an alkylene group having two or three carbon atoms is preferred]. "A" is more preferably a single bond or oxyethylene group. "A" is also preferably a group of Formula (2) wherein R⁷ is an ethylene group; and "n" is an integer of 0 to 5.

In Formula (1b), one of R³, R⁴, R⁵, and R⁶ represents a bond with "A" in Formula (1a), and the others each represent a hydrogen atom or an alkyl group having one to ten carbon atoms. Of R³, R⁴, R⁵, and R⁶, those representing hydrogen atom or an alkyl group having one to ten carbon atoms may be the same as or different from one another. Among them, hydrogen atom or methyl group is preferred, of which hydrogen atom is typically preferred.

Of compounds represented by Formula (1a), some compounds are combined with a group represented by Formula (1b) through R³ or R⁴ (combined at the 9-position of a 3,4-epoxytricyclo[5.2.1.0^{2,6}]decane skeleton), and the others are combined with the group through R⁵ or R⁶ (combined at the 8-position of the 3,4-epoxytricyclo[5.2.1.0^{2,6}]decane skeleton). A representative example of the former includes a compound represented by following Formula (1a-1), and a representative example of the latter includes a compound represented by following Formula (1a-2): 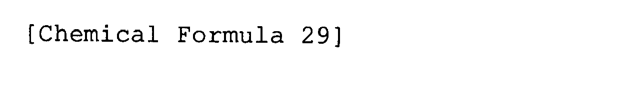 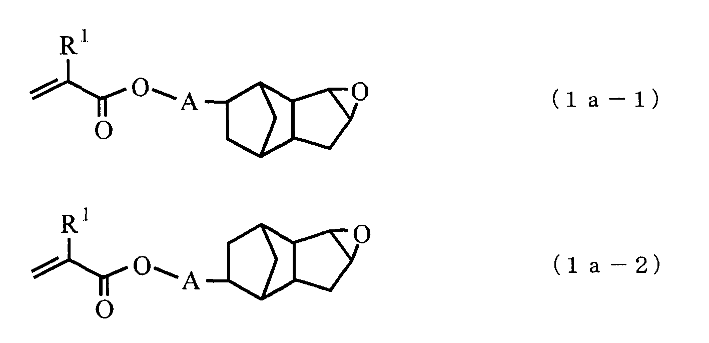 wherein R¹ represents a hydrogen atom or an alkyl group having one to seven carbon atoms which may be substituted with hydroxyl group; and "A" represents a single bond or a bivalent hydrocarbon group which may contain hetero atom.

Representative examples of such unsaturated carboxylic acid esters containing an 3,4-epoxytricyclo[5.2.1.0^{2,6}] decane skeleton, represented by Formula (1a) include epoxidized dicyclopentenyl (meth)acrylates [3,4-epoxytricyclo[5.2.1.0^{2,6}]dec-9-yl (meth)acrylates; and 3,4-epoxytricyclo[5.2.1.0^{2,6}]dec-8-yl(meth)acrylates], epoxidized dicyclopentenyloxyethyl (meth)acrylates [2-(3,4-epoxytricyclo[5.2.1.0^{2,6}]dec-9-yloxy)ethyl (meth)acrylates; and 2-(3,4-epoxytricyclo[5.2.1.0^{2,6}]dec-8-yloxy)ethyl (meth)acrylates], epoxidized dicyclopentenyloxybutyl (meth)acrylates, and epoxidized dicyclopentenyloxyhexyl (meth)acrylates.

The unsaturated carboxylic acid esters containing an 3,4-epoxytricyclo[5.2.1.0^{2,6} ]decane skeleton (cycloaliphatic epoxy (meth)acrylates and other cycloaliphatic epoxy unsaturated carboxylic acid esters) can each be synthetically prepared by epoxidizing an unsaturated carboxylic acid ester containing a tricyclo[5.2.1.0^{2,6}]dec-3-ene skeleton, represented by Formula (3a) (an alicyclic unsaturated carboxylic acid ester such as an alicyclic (meth)acrylate) using an epoxidizing agent such as peracetic acid or a hydroperoxide. By a process according to the present invention which will be illustrated in detail later, the double bond in the tricyclo[5.2.1.0^{2,6}]dec-3-ene skeleton can be epoxidized while maintaining substantially all of the unsaturated double bonds between carbons at the α-position and the β-position with respect to the carbonyl carbon of the unsaturated carboxylic acid ester.

Examples of the unsaturated carboxylic acid ester containing a tricyclo[5.2.1.0^{2,6}]dec-3-ene skeleton, represented by Formula (3a) used as a starting material include dicyclopentenyl (meth)acrylates [tricyclo[5.2.1.0^{2,6}]dec-3-en-9-yl (meth)acrylates; and tricyclo[5.2.1.0^{2,6}]dec-3-en-8-yl (meth)acrylates], dicyclopentenyloxyethyl (meth)acrylates [2-(tricyclo[5.2.1.0^{2,6}]dec-3-en-9-yloxy)ethyl (meth)acrylates; and 2-(tricyclo[5.2.1.0^{2,6}]dec-3-en-8-yloxy)ethyl (meth)acrylates], dicyclopentenyloxybutyl (meth)acrylates, and dicyclopentenyloxyhexyl (meth)acrylates.

Examples of products commercially available as these compounds include a product of Hitachi Chemical Co., Ltd., under the trade name of "FA-511A", wherein R¹ is hydrogen atom, and "A" is a single bond in Formula (3a) and wherein R³, R⁴, R⁵, and R⁶ in Formula (3b) are hydrogen atoms, except for one representing a bond with "A"; a product of Hitachi Chemical Co., Ltd., under the trade name of "FA-512A", wherein R¹ is hydrogen atom and "A" is oxyethylene group, and R³ , R⁴, R⁵, and R⁶ in Formula (3b) are hydrogen atoms, except for one representing a bond with "A"; and products of Hitachi Chemical Co., Ltd., under the trade names of "FA-512M" and "FA-512MT", wherein R¹ is methyl group and "A" is oxyethylene group, and wherein R³, R⁴, R⁵, and R⁶ in Formula (3b) are hydrogen atoms, except for one representing a bond with "A". Each of unsaturated carboxylic acid esters containing a tricyclo[5.2 .1.0^{2,6}]dec-3-ene skeleton can be used alone or in combination.

By carrying out epoxidation using an epoxidizing agent such as peracetic acid or a hydroperoxide, a double bond in the alicyclic structure can be epoxidized while maintaining substantially all the unsaturated bonds, such as (meth)acryloyl group, in an unsaturated carboxylic acid ester, in the preparation of an unsaturated carboxylic acid ester containing an 3,4-epoxytricyclo[5.2.1.0^{2,6}]decane skeleton.

A general polymerization inhibitor such as methoquinone (p-methoxyphenol), hydroquinone, or phenothiazine can be used in epoxidation. This is for the purpose of inhibiting polymerization of unsaturated bonds (double bonds between carbons at the α-position and β-position with respect to carbonyl carbon), such as (meth)acryloyl groups, in the starting material unsaturated carboxylic acid ester containing a tricyclo[5.2.1.0^{2,6}]dec-3-ene skeleton, represented by Formula (3a), and in the formed unsaturated carboxylic acid ester containing an 3,4-epoxytricyclo[5.2.1.0^{2,6}]decane skeleton, represented by Formula (1a). The amount of the polymerization inhibitor is not specifically limited and can be selected within the range of, for example, 0.03 to 5 parts by weight, and preferably 0.05 to 3 parts by weight, to 100 parts by weight of the starting material unsaturated carboxylic acid ester containing a tricyclo[5.2.1.0^{2,6}]dec-3-ene skeleton, represented by Formula (3a). An unsaturated carboxylic acid ester containing an 3,4-epoxytricyclo[5.2.1.0^{2,6}]decane skeleton, represented by Formula (1a) can be prepared as a result of the epoxidation of an unsaturated carboxylic acid ester containing a tricyclo[5.2.1.0^{2,6}]dec-3-ene skeleton, represented by Formula (3a). To prevent the polymerization, molecular oxygen is preferably used in combination with such a polymerization inhibitor.

Examples of epoxidizing agents for use in the present invention include peracids and hydroperoxides. Such peracids may be previously prepared, or prepared within the reaction system. Examples of peracids include organic peracids such as performic acid, peracetic acid, perbenzoic acid, and trifluoroperacetic acid. Among them, a peracid obtained by (air) oxidation of a corresponding aldehyde is preferably used. This is because a peracid prepared by this technique has a low moisture content of 0.8 percent by weight or less, frequently 0.6 percent by weight or less, and this contributes to a high conversion from an unsaturated carboxylic acid ester containing a tricyclo[5.2.1.0^{2,6}]dec-3-ene skeleton, represented by Formula (3a) to an unsaturated carboxylic acid ester containing an 3,4-epoxytricyclo[5.2.1.0^{2,6}]decane skeleton, represented by Formula (1a). In particular, peracetic acid obtained by oxidation of acetaldehyde with air or oxygen is typically preferably used, because such peracetic acid is industrially inexpensively available and is highly stable. Such peracetic acid having a low moisture content can be prepared, for example, according to the processes described in German Unexamined Patent Application Publication No. 1418465 and Japanese Unexamined Patent Application Publication (JP-A) No. 54-3006.

Examples of hydroperoxides include hydrogen peroxide, tert-butyl hydroperoxide, and cumene peroxide. An epoxidation technique using hydrogen peroxide in combination with a phase transfer catalyst and a metal salt of tungstic acid can also be employed. Examples of such phase transfer catalysts include trialkylammonium salts such as trioctylmethylammonium chloride, trioctylmethylammonium sulfate, tetrahexylammonium sulfate, and trihexylmethylammonium sulfate. The amount of the phase transfer catalyst is 0.1 to 20 parts by weight, preferably 0.5 to 18 parts by weight, and more preferably 1 to 15 parts by weight, to 100 parts by weight of hydrogen peroxide. An example of the metal salt of tungstic acid includes sodium tungstate. The amount of the metal salt of tungstic acid is not specifically limited, and is, for example, 1 to 30 parts by weight, preferably 2 to 18 parts by weight, and more preferably 3 to 15 parts by weight, to 100 parts by weight of hydrogen peroxide. A reaction can more quickly proceed by further using phosphoric acid or a salt thereof, aminomethylsulfonic acid, and/or sodium sulfate. The amount of these components can be selected, for example, within the range of 1 to 80 parts by weight, and preferably 5 to 75 parts by weight, to 100 parts by weight of the metal salt of tungstic acid. If the amount of the phase transfer catalyst and the metal salt of tungstic acid is out of the above-specified range, an appropriate epoxidation may not proceed. Instead of a metal salt of tungstic acid, molybdic acid or a metal salt of molybdic acid can be used. According to this technique, a reaction can be carried out in a two-phase system including water and an organic solvent.

Each of these epoxidizing agents can be used alone or in combination selectively. The amount of epoxidizing agents may be appropriately selected according typically to the type of epoxidizing agents to be used, the desired degree of epoxidation, and the type of the unsaturated carboxylic acid ester containing a tricyclo[5.2.1.0^{2,6}]dec-3-ene skeleton to be used.

Catalysts can be used in epoxidation according to necessity. An alkali (base) or an acid can be used as a catalyst when a peracid, for example, is used as an epoxidizing agent. The alkali includes, for example, sodium carbonate; and the acid includes, for example, sulfuric acid and organic acids. A mixture of tungstic acid and sodium hydroxide can be used as a catalyst when a hydroperoxide such as hydrogen peroxide is used as an epoxidizing agent. More specifically, a catalytic effect can be obtained by the combination use of an organic acid with hydrogen peroxide or molybdenum hexacarbonyl with tert-butyl hydroperoxide. Epoxidation can also be carried out using an alkali metal salt of tungstic acid in combination with phosphoric acid or a phosphoric acid ester , or an alkylammonium with hydrogen peroxide.

The molar ratio of the epoxidizing agent to the unsaturated carboxylic acid ester containing a tricyclo[5.2.1.0^{2,6}]dec-3-ene skeleton, represented by Formula (3a) is preferably such that the moles of the epoxidizing agent is equal to or more than the moles of double bonds in the alicyclic skeleton. It is, however, generally disadvantageous that the molar ratio exceeds 2 times by mole from the viewpoints of economical efficiency and occurrence of side reactions. For example, the amount of peracetic acid, if used as the epoxidizing agent, may be generally selected from 1 time by mole or more, and preferably 1 to 1.5 times by mole.

Epoxidation may be carried out in the presence of a solvent. Such a solvent is used for the purpose typically of stabilizing the system by reducing the viscosity of materials, and diluting the epoxidizing agent. Inert solvents are preferably used as the solvent. Examples of such inert solvents include aromatic hydrocarbons such as benzene, toluene, and xylenes; aliphatic or alicyclic hydrocarbons such as hexane and cyclohexane; halogenated hydrocarbons such as carbon tetrachloride and chloroform; esters such as ethyl acetate; and ethers such as diethyl ether and 1,4-dioxane. When peracetic acid is used as the epoxidizing agent, the solvent is preferably an aromatic hydrocarbon, an ether, or an ester.

The temperature upon epoxidation is preferably 0°C to 70°C when peracetic acid is used as the epoxidizing agent. If epoxidation is carried out at a temperature of 0°C or lower, the reaction may proceed slowly, and if it is carried out at a temperature of 70°C, peracetic acid may decompose. For the same reasons, the temperature is preferably 20°C to 150°C when tert-butyl hydroperoxide as a hydroperoxide is used in combination with a molybdenum dioxide diacetylacetonate compound. An extra procedure is not required during the reaction. It is enough to stir a reaction mixture, for example, for one to eight hours. This yields an unsaturated carboxylic acid ester containing an 3,4-epoxytricyclo[5.2.1.0^{2,6}]decane skeleton according to the present invention.

After the composition of the reaction, an unsaturated carboxylic acid ester containing an 3,4-epoxytricyclo[5.2.1.0^{2,6}]decane skeleton can be isolated from the reaction mixture, for example, by precipitating in a poor solvent, by placing the reaction mixture into hot water with stirring, and removing the solvent by distillation, or by direct desolvation.

Unsaturated carboxylic acid esters containing an 3,4-epoxytricyclo[5.2.1.0^{2,6}]decane skeleton, represented by Formula (1a) according to the present invention can be used as monomers for various functional polymers. Polymers having monomeric units corresponding to the unsaturated carboxylic acid esters containing an 3,4-epoxytricyclo[5.2.1.0^{2,6}]decane skeleton, represented by Formula (1a) are useful as curable resins and radiation-sensitive resins. As monomers for functional polymers, epoxidized dicyclopentenyl (meth)acrylates and epoxidized dicyclopentenyloxyethyl (meth)acrylates are especially preferred.

Next, a copolymer according to the present invention will be illustrated in detail below. A monomeric unit (A) containing an alkali-soluble group acts to impart alkali solubility to the polymer. This allows the polymer to be dissolved in an alkali aqueous solution (developer) upon development. The monomeric unit (A) also acts to cure the polymer as a result of crosslinking using a crosslinking agent or a reaction of a cyclic ether-containing group such as epoxy group or oxetane ring in the molecule of the polymer upon light exposure, to thereby impart a necessary hardness (rigidity) as a resist to a film (coat) and to make the polymer insoluble in alkali. Such copolymers according to the present invention are typically useful as resins for negative-working photoresists.

A monomeric unit (A) containing an alkali-soluble group can be introduced into a polymer by subjecting a polymerizable unsaturated compound (a) having an alkali-soluble group to copolymerization. The alkali-soluble group can be any group generally used in the field of resists and includes, for example, carboxyl group and phenolic hydroxyl group. Representative examples of the polymerizable unsaturated compound (a) having an alkali-soluble group include, but are not limited to, unsaturated carboxylic acids or acid anhydrides thereof, and hydroxystyrenes or derivatives thereof. Among them, unsaturated carboxylic acids and acid anhydrides thereof are preferred.

Examples of unsaturated carboxylic acids and acid anhydrides thereof include α,β-unsaturated carboxylic acids such as acrylic acid, methacrylic acid, itaconic acid, crotonic acid, maleic acid, and fumaric acid; and acid anhydrides thereof such as maleic anhydride and itaconic anhydride. Among them, acrylic acid and methacrylic acid are typically preferred. Each of these polymerizable unsaturated compounds (a) having an alkali-soluble group can be used alone or in combination.

The content of the monomeric units (A) containing an alkali-soluble group in the copolymer varies depending on the types of monomers to be used, and the type of the resist (negative-working or positive-working), and is generally 10 to 50 percent by weight, preferably 12 to 40 percent by weight, and more preferably 14 to 30 percent by weight of total monomeric units constituting the copolymer. If the content is excessively small, the copolymer may be resistant to dissolution in an alkali developer and show poor developability. In contrast, if it is excessively large, etching resistance after development may deteriorate.

The monomeric unit (B) corresponding to an epoxy-containing polymerizable unsaturated compound acts to cure the polymer as a result of crosslinking using a crosslinking agent or a reaction with an alkali-soluble group, such as carboxyl group or phenolic hydroxyl group, in the molecule of the polymer upon light exposure, to thereby impart a necessary hardness (rigidity) as a resist to a film (coat), to increase the etching resistance, and to make the polymer insoluble in alkali.

A monomeric unit (B) corresponding to an epoxy-containing polymerizable unsaturated compound can be introduced into a polymer by subjecting an epoxy-containing polymerizable unsaturated compound (b) to copolymerization. The epoxy-containing polymerizable unsaturated compound (b) is preferably a compound containing a group having an unsaturated bond and a polycyclic aliphatic group having an epoxy group on its ring. Examples of the polycyclic aliphatic group include dicyclopentyl group and tricyclodecyl group. An example of the unsaturated bond includes carbon-carbon double bond, and examples of the group having an unsaturated bond include vinyl group, allyl group, methallyl group, acryloyl group, and methacryloyl group.

At least one unsaturated carboxylic acid ester containing an 3,4-epoxytricyclo[5.2.1.0^{2,6}]decane skeleton, represented by Formula (1a) is used as an epoxy-containing polymerizable unsaturated compound (b) in the present invention. For example, each of a compound represented by Formula (1a-1) and a compound represented by Formula (1a-2) can be used alone or in combination in optional proportions. If the two compounds are used in combination, the ratio of the compound of Formula (1a-1) to the compound of Formula (1a-2) is preferably 5:95 to 95:5, more preferably 10:90 to 90:10, and further preferably 20:80 to 80:20.

According to the present invention, one or more unsaturated carboxylic acid esters containing an 3,4-epoxytricyclo[5.2.1.0^{2,6}]decane skeleton, represented by Formula (1a) can be used in combination with one or more other epoxy-containing polymerizable unsaturated compounds (hereinafter also referred to as "other epoxy-containing polymerizable unsaturated compound(s)"). Examples of the other epoxy-containing polymerizable unsaturated compounds include polymerizable unsaturated compounds containing an oxirane ring (single ring), including (meth)acrylic acid ester derivatives, such as oxiranyl (meth)acrylates, glycidyl (meth)acrylates, 2-methylglycidyl (meth)acrylates, 2-ethylglycidyl (meth)acrylates, 2-oxiranylethyl (meth)acrylates, 2-glycidyloxyethyl (meth)acrylates, 3-glycidyloxypropyl (meth)acrylates, and glycidyloxyphenyl (meth)acrylates; polymerizable unsaturated compounds containing an epoxy-containing alicyclic carbon ring such as an 3,4-epoxycyclohexane ring (including (meth)acrylic acid ester derivatives), such as 3,4-epoxycyclohexyl (meth)acrylates, 3,4-epoxycyclohexylmethyl (meth)acrylates, 2-(3,4-epoxycyclohexyl)ethyl (meth)acrylates, 2-(3,4-epoxycyclohexylmethyloxy)ethyl (meth)acrylates, and 3-(3,4-epoxycyclohexylmethyloxy)propyl (meth)acrylates; and polymerizable unsaturated compounds containing an 5,6-epoxy-2-bicyclo[2.2.1]heptane ring (including (meth)acrylic acid ester derivatives), such as 5,6-epoxy-2-bicyclo[2.2.]heptyl (meth)acrylates. The other epoxy-containing polymerizable unsaturated compounds usable herein also include epoxy-containing vinyl ether compounds and epoxy-containing allyl ether compounds.

The content of the monomeric units (B) corresponding to an epoxy-containing polymerizable unsaturated compound in the copolymer is 40 to 90 percent by weight, preferably 45 to 85 percent by weight, and more preferably 50 to 80 percent by weight of total monomeric units. If the content is less than 40 percent by weight, a crosslinking reaction upon exposure may not sufficiently proceed, and this may cause poor thermal stability and deteriorated etching resistance. In contrast, if the content exceeds 90 percent by weight, sufficient solubility in alkali may not be obtained to thereby fail to carry out patterning satisfactorily.

The total content of monomeric units corresponding to unsaturated carboxylic acid esters containing an 3,4-epoxytricyclo[5.2.1.0^{2,6}]decane skeleton, represented by Formula (1a) in the monomeric units (B) corresponding to epoxy-containing polymerizable unsaturated compounds may be 30 percent by weight or more in the present invention. If the ratio is less than 30 percent by weight, the resulting polymer may exhibit insufficient performance required as a radiation-sensitive resin. For example, when used in combination with a polymerizable unsaturated compound containing an epoxy-containing alicyclic carbon ring, the polymer may show insufficient storage stability. When used in combination with a polymerizable unsaturated compound containing an oxirane ring (single ring), such as a glycidyl group-containing monomer, the polymer may have insufficient thermal stability.

A monomeric unit (C) for use in the present invention is a monomeric unit corresponding to at least one epoxy-free polymerizable unsaturated compound selected from the group consisting of (c1) alkyl-substituted or -unsubstituted styrenes, (c2) unsaturated carboxylic acid esters represented by Formula (4), and (c3) N-substituted maleimides. The monomeric unit (C) acts to impart hardness (rigidity) necessary as a resist to the film (coat). In addition, the corresponding monomer acts to allow a copolymerization reaction to smoothly proceed. Some monomeric units (C) also act to increase the hardness (rigidity) of the film (coat) as a result typically of a crosslinking reaction.

Examples of alkyl groups in the alkyl-substituted or - unsubstituted styrenes (c1) include alkyl groups having about one to about seven carbon atoms, such as methyl, ethyl, propyl, isopropyl, butyl, isobutyl, t-butyl, and hexyl groups. Among them, alkyl groups having one to four carbon atoms, such as methyl group and ethyl group, are preferred, of which methyl group is typically preferred. Such an alkyl group may be bound to either of vinyl group and benzene ring of styrene. Representative examples of alkyl-substituted or -unsubstituted styrenes (c1) include styrene, α-methylstyrene, and vinyltoluenes (o-vinyltoluene, m-vinyltoluene, and p-vinyltoluene). Each of these can be used alone or in combination.

Examples of the alkyl group having one to seven carbon atoms as R⁸ in unsaturated carboxylic acid esters (c2) represented by Formula (4) include methyl, ethyl, propyl, isopropyl, butyl, isobutyl, t-butyl, and hexyl groups. R⁸ is typically preferably hydrogen atom or methyl group.

Examples of the primary or secondary alkyl group having one to twelve carbon atoms as R⁹ include methyl, ethyl, propyl, isopropyl, butyl, isobutyl, s-butyl, hexyl, octyl, decyl, and dodecyl groups. Examples of the alkenyl group having two to twelve carbon atoms include primary or secondary alkenyl groups such as allyl, 3-butenyl, and 5-hexenyl groups. The aryl group includes, for example, phenyl group. The aralkyl group includes, for example, benzyl group. Examples of the group containing a cyclic ether having four or more members include groups having a cyclic ether structure such as oxetane ring, oxolane ring, oxane ring, or oxepane ring. Examples of oxetane ring-containing groups include oxetanyl group, 3-methyl-3-oxetanyl group, 3-ethyl-3-oxetanyl group, (3-methyl-3-oxetanyl)methyl group, (3-ethyl-3-oxetanyl)methyl group, 2-(3-methyl-3-oxetanyl)ethyl group, 2-(3-ethyl-3-oxetanyl)ethyl group, 2-[(3-methyl-3-oxetanyl)methyloxy]ethyl group, 2-[(3-ethyl-3-oxetanyl)methyloxy]ethyl group, 3-[(3-methyl-3-oxetanyl)methyloxy]propyl group, and 3-[(3-ethyl-3-oxetanyl)methyloxy]propyl group. Examples of oxolane ring-containing groups include tetrahydrofurfuryl group, 3-oxolanyl group, 3-methyl-3-oxolanyl group, 3-ethyl-3-oxolanyl group, (2-methyl-2-oxolanyl)methyl group, (2-ethyl-2-oxolanyl)methyl group, 2-(2-methyl-2-oxolanyl)ethyl group, 2-(2-ethyl-2-oxolanyl)ethyl group, 2-[(2-methyl-2-oxolanyl)methyloxy]ethyl group, 2-[(2-ethyl-2-oxolanyl)methyloxy]ethyl group, 3-[(2-methyl-2-oxolanyl)methyloxy]propyl group, and 3-[(2-ethyl-2-oxolanyl)methyloxy]propyl group.

In the -(R¹⁰-O)ₘ-R¹¹ group as R⁹, R¹⁰ represents a bivalent hydrocarbon group having one to twelve carbon atoms; R¹¹ represents a hydrogen atom or a hydrocarbon group; and "m" represents an integer of 1 or more. Examples of the bivalent hydrocarbon group having one to twelve carbon atoms are as with the bivalent hydrocarbon groups having one to twelve carbon atoms in R⁶. Among them, preferred are alkylene groups having two to six carbon atoms, such as ethylene, propylene, tetramethylene, and hexamethylene groups; and alicyclic hydrocarbon groups having three to six members, such as cyclohexylene group. Examples of the hydrocarbon group as R¹¹ include aliphatic hydrocarbon groups including alkyl groups such as methyl, ethyl, propyl, isopropyl, butyl, and hexyl groups, of which alkyl groups having one to six carbon atoms are preferred; alicyclic hydrocarbon groups including cycloalkyl groups such as cyclopentyl group and cyclohexyl group, and bridged carbocyclic groups such as norbornyl group (bicyclo[2.2.1]heptyl group) and tricyclo[5.2 .1.0^{2,6}]decyl group; and groups each containing two or more of these combined with each other. The repetition number "m" is preferably an integer of 1 to 10, more preferably an integer of 1 to 4, and typically preferably 1.

Representative examples of the unsaturated carboxylic acid esters (c2) represented by Formula (4) include methyl (meth)acrylates, ethyl (meth)acrylates, propyl (meth)acrylates, isopropyl (meth)acrylates, butyl (meth)acrylates, allyl (meth)acrylates, phenyl (meth)acrylates, benzyl (meth)acrylates, oxetanyl (meth)acrylates, 3-methyl-3-oxetanyl (meth)acrylates, 3-ethyl-3-oxetanyl (meth)acrylates, (3-methyl-3-oxetanyl)methyl (meth)acrylates, (3-ethyl-3-oxetanyl)methyl (meth)acrylates, 2-(3-methyl-3-oxetanyl)ethyl (meth)acrylates, 2-(3-ethyl-3-oxetanyl)ethyl (meth)acrylates, 2-[(3-methyl-3-oxetanyl)methyloxy]ethyl (meth)acrylates, 2-[(3-ethyl-3-oxetanyl)methyloxy]ethyl (meth)acrylates, 3-[(3-methyl-3-oxetanyl)methyloxy]propyl (meth)acrylates, 3-[(3-ethyl-3-oxetanyl)methyloxy]propyl (meth)acrylates, 2-hydroxyethyl (meth)acrylates, tetrahydrofurfuryl (meth)acrylates, 2-hydroxypropyl (meth)acrylates, and 2-(tricyclo[5.2.1.0^{2,6}]decyloxy)ethyl (meth)acrylates. Each of the unsaturated carboxylic acid esters (c2) represented by Formula (1) can be used alone or in combination.

An example of the N-substituted maleimides (c3) includes a compound represented by following Formula (6): 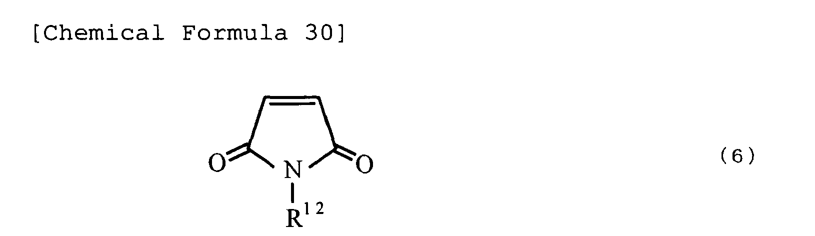 wherein R¹² represents an organic group.

Such organic groups include hydrocarbon groups and heterocyclic groups. Examples of the hydrocarbon groups include aliphatic hydrocarbon groups including alkyl groups such as methyl, ethyl, propyl, isopropyl, butyl, and hexyl groups, of which alkyl groups having one to six carbon atoms are preferred; alicyclic hydrocarbon groups including cycloalkyl groups such as cyclopentyl group, cyclohexyl group, and cyclooctyl group, and bridged carbocyclic groups such as adamantyl group and norbornyl group; aryl groups such as phenyl group; aralkyl groups such as benzyl group; and groups each containing two or more of these combined with each other. Examples of the heterocyclic groups include non-aromatic or aromatic heterocyclic groups having about five to about ten members and containing at least one hetero atom selected from the group consisting of nitrogen atom, oxygen atom, and sulfur atom.

Representative examples of the N-substituted maleimides (c3) include N-cycloalkylmaleimides such as N-cyclopentylmaleimide, N-cyclohexylmaleimide, and N-cyclooctylmaleimide; N-(bridged carbocyclic group)-substituted maleimides such as N-adamantylmaleimide and N-norbornylmaleimide; N-alkylmaleimides such as N-methylmaleimide, N-ethylmaleimide, and N-propylmaleimide; N-arylmaleimides such as N-phenylmaleimide; and N-aralkylmaleimides such as N-benzylmaleimide. Among them, preferred are N-cycloalkylmaleimides such as N-cyclohexylmaleimide, and N-(bridged carbocyclic group)-substituted maleimides. Each of the N-substituted maleimides (c3) can be used alone or in combination.

A copolymer according to the present invention may further contain a small amount of other monomeric units in addition to the monomeric units (A), (B), and (C). Examples of the other monomeric units include units corresponding to (meth)acrylamide and (meth)acrylonitrile. When a copolymer according to the present invention contains the monomeric units (A) and (B) but does not contain the monomeric unit (C), the total amount of the monomeric units (A) and (B) is generally 98 percent by weight or more, preferably 99 percent by weight or more, and more preferably substantially 100 percent by weight of the total monomeric units. When a copolymer according to the present invention contains the monomeric units (A), (B), and (C), the total amount of the three monomeric units is, for example, 90 percent by weight or more, preferably 95 percent by weight or more, more preferably 98 percent by weight or more, and especially preferably substantially 100 percent by weight of the total monomeric units.

A copolymer according to the present invention can be prepared by subjecting a monomer mixture to copolymerization, in which the monomer mixture is a monomer mixture containing the polymerizable unsaturated compound (a) having an alkali-soluble group, and the epoxy-containing polymerizable unsaturated compound (b), or is a monomer mixture containing, in addition to the compounds (a) and (b), the epoxy-free polymerizable unsaturated compound (c), the epoxy-containing polymerizable unsaturated compound (b) occupies 40 to 90 percent by weight of the total monomers, and 30 percent by weight or more of the epoxy-containing polymerizable unsaturated compound (b) is an unsaturated carboxylic acid ester containing an 3,4-epoxytricyclo[5.2.1.0^{2,6}]decane skeleton, represented by Formula (2a) and/or (2b).

A polymerization initiator for use in copolymerization can be a regular radical polymerization initiator, and examples thereof include azo compounds such as 2,2'-azobisisobutyronitrile, 2,2'-azobis(2,4-dimethylvaleronitrile), 2,2'-azobis(4-methoxy-2,4-dimethylvaleronitrile), dimethyl 2,2'-azobis(2-methylpropionate), diethyl 2,2'-azobis(2-methylpropionate), and dibutyl 2,2'-azobis(2-methylpropionate); organic peroxides such as benzoyl peroxide, lauroyl peroxide, t-butyl peroxypivalate, and 1,1-bis(t-butylperoxy)cyclohexane; and hydrogen peroxide. A peroxide, if used as a radical polymerization initiator, may be used in combination with a reducing agent to constitute a redox initiator. Among these initiators, azo compounds are preferred, of which 2,2'-azobisisobutyronitrile, 2,2'-azobis (2,4-dimethylvaleronitrile), and dimethyl 2,2'-azobis(2-methylpropionate) are typically preferred.

The amount of the polymerization initiator can be appropriately selected within a range not adversely affecting smooth copolymerization and is generally about 1 to about 10 percent by weight and preferably about 2 to about 8 percent by weight, to the total amount of monomers (total monomeric components) and the polymerization initiator.

Copolymerization can be carried out according to a procedure generally used in related art for preparing styrenic polymers and acrylic polymers. Examples of such procedures include solution polymerization, bulk polymerization, suspension polymerization, bulk-suspension polymerization, and emulsion polymerization. Among them, solution polymerization is preferred. The monomers and the polymerization initiator may each be fed to the reaction system in one portion, or part or all of them may be added dropwise to the reaction system. Examples of procedures usable herein include a process of carrying out polymerization by dissolving a polymerization initiator in a polymerization solvent to yield a solution, and adding the solution dropwise to a mixture of monomers and the polymerization solvent held at a constant temperature; and a process of carrying out polymerization by dissolving monomers and a polymerization initiator in a polymerization solvent to yield solutions, respectively, and adding the solutions dropwise to a polymerization solvent held at a constant temperature (dropping polymerization).

The polymerization solvent can be selected as appropriate according typically to the monomer composition. Examples of such polymerization solvents include ethers including open-chain ethers such as diethyl ether, 3-methoxy-1-butanol, propylene glycol monomethyl ether, diethylene glycol ethyl methyl ether, and other glycol ethers, and cyclic ethers such as tetrahydrofuran and dioxane; esters including methyl acetate, ethyl acetate, butyl acetate, ethyl lactate, 3-methoxybutyl acetate, ethyl 3-ethoxypropionate, and glycol ether esters such as propylene glycol monomethyl ether acetate; ketones such as acetone, methyl ethyl ketone, methyl isobutyl ketone, and cyclohexanone; amides such as N,N-dimethylacetamide and N,N-dimethylformamide; sulfoxides such as dimethyl sulfoxide; alcohols such as methanol, ethanol, and propanol; hydrocarbons including aromatic hydrocarbons such as benzene, toluene, and xylenes, aliphatic hydrocarbons such as hexane, and alicyclic hydrocarbons such as cyclohexane; and mixtures of these solvents. The polymerization temperature can be selected as appropriate within a range of, for example, about 30°C to about 150°C.

By carrying out the process, a copolymer according to the present invention is formed. The number-average molecular weight of the copolymer is, for example, about 3000 to about 50000, preferably about 3500 to about 40000, and more preferably about 4000 to about 30000. The molecular weight distribution [(weight-average molecular weight Mw)/(number-average molecular weight Mn)] of the copolymer is about 1 to about 3.

A polymerization mixture obtained by the above-mentioned process can be used as a radiation-sensitive resin composition such as a resin composition for a photoresist, after adjusting the solid content of the mixture and/or filtering the mixture according to necessity, and adding appropriate additives such as a light-activatable acid generator, a crosslinking agent (for a negative-working resist), a resin, and/or a colorant. A polymer formed as a result of the polymerization can also be used as a radiation-sensitive resin composition such as a resin composition for a photoresist, by purifying the polymer typically by precipitation or reprecipitation, and dissolving the purified polymer together with the appropriate additives in a solvent such as a solvent for a resist. The additives such as a light-activatable acid generator and a crosslinking agent, and the resist solvent can be selected depending typically on the monomer composition and can be those generally used in the fields of resists.

A solvent for use in the precipitation or reprecipitation of the polymer can be any of organic solvents, water, and mixtures of organic solvents and water. Examples of organic solvents include hydrocarbons including aliphatic hydrocarbons such as pentane, hexane, heptane, and octane, alicyclic hydrocarbons such as cyclohexane and methylcyclohexane, and aromatic hydrocarbons such as benzene, toluene, and xylenes; halogenated hydrocarbons including halogenated aliphatic hydrocarbons such as methylene chloride, chloroform, and carbon tetrachloride, and halogenated aromatic hydrocarbons such as chlorobenzene and dichlorobenzenes; nitro compounds such as nitromethane and nitroethane; nitriles such as acetonitrile and benzonitrile; ethers including open-chain ethers such as diethyl ether, diisopropyl ether, and dimethoxyethane, and cyclic ethers such as tetrahydrofuran and dioxane; ketones such as acetone, methyl ethyl ketone, and diisobutyl ketone; esters such as ethyl acetate and butyl acetate; carbonates such as dimethyl carbonate, diethyl carbonate, ethylene carbonate, and propylene carbonate; alcohols such as methanol, ethanol, propanol, isopropyl alcohol, and butanol; carboxylic acids such as acetic acid; and mixtures containing these solvents.

### EXAMPLES

The present invention will be illustrated in further detail with reference to several examples below, which, however, by no means limit the scope of the present invention. All percentages are by weight, unless otherwise specified.

### [Preparation of unsaturated carboxylic acid ester containing an 3,4-epoxytricyclo[5.2.1.0^{2,6}]decane skeleton]

### Example 1

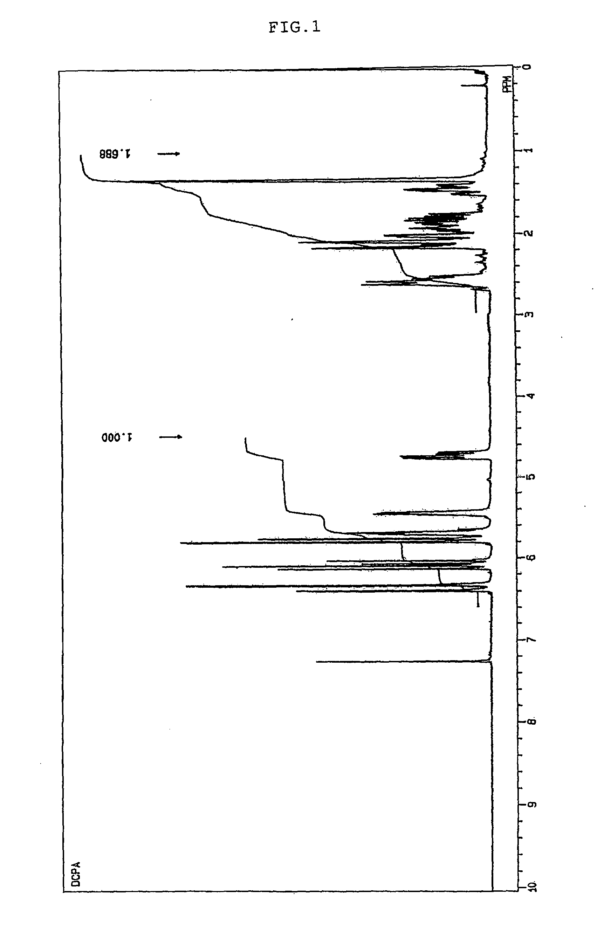

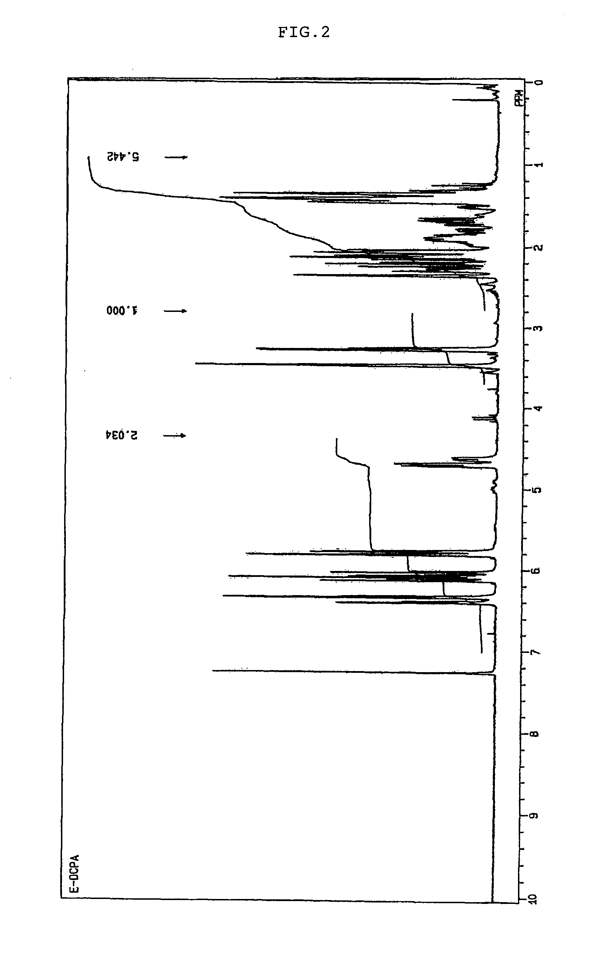

In a jacketed 1-liter flask were placed 100 g of tricyclo[5.2 .1.0^{2,6}]dec-3-enyl acrylate (a product of Hitachi Chemical Co., Ltd. under the trade name of "FA-511A", having a molecular weight of 204.3) and 100 g of ethyl acetate. The temperature within the reaction system was adjusted to 50°C while blowing air thereinto, and 156 g of a solution of peracetic acid in ethyl acetate having a peracetic acid concentration of 29.6% and a moisture content of 0.2% was added dropwise over about one hour. After the completion of dropwise addition of peracetic acid, the mixture was aged at 50°C for four hours, and the reaction was completed. The crude reaction mixture was washed with water at 50°C, from which low-boiling components were removed at 70°C/10 mmHg, to thereby yield 91.8 g of 3,4-epoxytricyclo[5.2.1.0^{2,6}]decyl acrylate. This had an oxirane-oxygen concentration of 6.74% and a viscosity of 61 cP at 25°C. This was subjected to ¹H-NMR measurement to find that a peak derived from an internal double bond at δ of 5.5 to 5.8 ppm as observed in the chart in FIG. 1 substantially disappears, and a proton peak derived from epoxy group occurs at δ of about 3.2 to 3.6 ppm in the chart in FIG. 2.

### Example 2

In a jacketed 1-liter flask were placed 100 g of tricyclo[5.2.1.0^{2,6}]dec-3-enyl acrylate (a product of Hitachi Chemical Co., Ltd. under the trade name of "FA-511A", having a molecular weight of 204.3), 100 g of toluene, 0.24 g of phosphoric acid, 4.65 g of trimethyloctylammonium chloride, and 6.60 g of sodium tungstate. The temperature within the reaction system was adjusted to 30°C while blowing air thereinto, and 83.5 g of 30% hydrogen peroxide was added dropwise over about one hour. After the completion of dropwise addition of hydrogen peroxide, the mixture was aged at 40°C for two hours, and the reaction was completed. The crude reaction mixture was washed with water at 40°C, from which low-boiling components were removed at 70°C/10 mmHg, to thereby yield 91.6 g of 3,4-epoxytricyclo[5.2.1.0^{2,6}]decyl acrylate. This had an oxirane-oxygen concentration of 6.62% and a viscosity of 103 cP at 25°C. This was subjected to ¹H-NMR measurement to find that a peak derived from an internal double bond at δ of 5.5 to 5.8 ppm as observed in the chart in FIG. 1 substantially disappears, and a proton peak derived from epoxy group occurs at δ of about 3.2 to 3.6 ppm in the chart in FIG. 2.

### Example 3

In a jacketed 1-liter flask were placed 100 g of 2-(tricyclo[5.2.1.0^{2,6}]dec-3-enyl)oxyethyl methacrylate (a product of Hitachi Chemical Co., Ltd. under the trade name of "FA-512MT", having a molecular weight of 262) and 20 g of ethyl acetate. The temperature within the reaction system was adjusted to 50°C while blowing air thereinto, and 106.4 g of a solution of peracetic acid in ethyl acetate having a peracetic acid concentration of 29.6% and a moisture content of 0.2% was added dropwise over about one hour. After the completion of dropwise addition of peracetic acid, the mixture was aged at 50°C for four hours, and the reaction was completed. The crude reaction mixture was washed with water at 50°C, from which low-boiling components were removed at 70°C/10 mmHg, to thereby yield 96.0 g of 2-(3,4-epoxytricyclo[5.2.1.0^{2,6}]decyloxy)ethyl methacrylate. This had an oxirane-oxygen concentration of 5.20% and a viscosity of 144 cP at 25°C. This was subjected to ¹H-NMR measurement to find that a peak derived from an internal double bond at δ of 5.5 to 5.8 ppm as observed in the chart in FIG. 1 substantially disappears, and a proton peak derived from epoxy group occurs at δ of about 3.2 to 3.6 ppm in the chart in FIG. 2.

### Example 4

In a jacketed 1-liter flask were placed 100 g of 2-(tricyclo[5.2.1.0^{2,6}]dec-3-enyloxy)ethyl acrylate (a product of Hitachi Chemical Co., Ltd. under the trade name of "FA-512A", having a molecular weight of 248) and 50 g of ethyl acetate. The temperature within the reaction system was adjusted to 50°C while blowing air thereinto, and 119.5 g of a solution of peracetic acid in ethyl acetate having a peracetic acid concentration of 29.5% and a moisture content of 0.2% was added dropwise over about one hour. After the completion of dropwise addition of peracetic acid, the mixture was aged at 50°C for four hours, and the reaction was completed. The crude reaction mixture was washed with water at 50°C, from which low-boiling components were removed at 70°C/10 mmHg, to thereby yield 88.4 g of 2-(3,4-epoxytricyclo[5.2.1.0^{2,6}]decyl) acrylate. This had an oxirane-oxygen concentration of 5.67% and a viscosity of 160 cP at 25°C. This was subjected to ¹H-NMR measurement to find that a peak derived from an internal double bond at δ of 5.5 to 5.8 ppm as observed in the chart in FIG. 1 substantially disappears, and a proton peak derived from epoxy group occurs at δ of about 3.2 to 3.6 ppm in the chart in FIG. 2.

### [Preparation of copolymers having monomeric unit corresponding to unsaturated carboxylic acid ester containing 3,4-epoxytricyclo[5.2.1.0^{2,6}]decane skeleton]

Next, a process for the preparation of a copolymer according to the present invention will be illustrated in further detail with reference to several examples below. The viscosities of copolymer solutions and radiation-sensitive resin composition solutions were determined with a Brookfield viscometer. The weight-average molecular weights (in terms of polystyrene) and degrees of dispersion [(weight-average molecular weight Mw)/(number-average molecular weight Mn)] of copolymers were measured with a gel permeation chromatography (GPC) system available from Shimadzu Corporation under the trade name of "K2479". The analyses were carried out under the following conditions.
Column: SHIMADU Shim-pack GPC-80M
Eluent: tetrahydrofuran (THF) 1 ml/min
Temperature (oven): 40°C
Detector: refractive index (RI) detector

### Example 5

The inside of a 1-liter flask equipped with a reflux condenser, dropping funnels, and a stirrer was allowed to be a nitrogen atmosphere by feeding a suitable amount of nitrogen thereto; 200 parts by weight of 3-methoxy-1-butanol and 105 parts by weight of 3-methoxybutyl acetate were placed in the flask and were heated to 70°C with stirring. Next, a solution was prepared by dissolving 55 parts by weight of methacrylic acid (MAA), 175 parts by weight of a 50:50 (by mole) mixture (E-DCPA) of 3,4-epoxytricyclo[5.2.1.0^{2,6}]dec-9-yl acrylate (belonging to compounds represented by Formula (1a-1)) and 3,4-epoxytricyclo[5.2.1.0^{2,6}]dec-8-yl acrylate (belonging to compounds represented by Formula (1a-2)), and 70 parts by weight of N-cyclohexylmaleimide (CHMI) in 140 parts by weight of 3-methoxybutyl acetate. The solution was added dropwise into the flask using a dropping pump over about four hours. Separately, 30 parts by weight of 2,2'-azobis(2,4-dimethylvaleronitrile) as a polymerization initiator was dissolved in 225 parts by weight of 3-methoxybutyl acetate to yield a solution, and this solution was added dropwise into the flask using another dropping pump over about five hours. After the completion of dropwise addition of the polymerization initiator, the mixture was held at the same temperature for about four hours, then cooled to room temperature, and thereby yielded a copolymer solution having a viscosity at 23°C of 114 mPa.s, a solid content of 32.6 percent by weight, and an acid value as a solution of 34.3 mg-KOH/g. The formed copolymer had a weight-average molecular weight (Mw) of 13600 and a molecular weight distribution of 2.54.

### Example 6

The inside of a 1-liter flask equipped with a reflux condenser, dropping funnels, and a stirrer was allowed to be a nitrogen atmosphere by feeding a suitable amount of nitrogen thereto; 150 parts by weight of 3-methoxy-1-butanol and 110 parts by weight of 3-methoxybutyl acetate were placed in the flask; and the mixture was heated to 70°C with stirring. Next, a solution was prepared by dissolving 60 parts by weight of methacrylic acid (MAA) and 240 parts by weight of a 50:50 (by mole) mixture (E-DCPA) of 3,4-epoxytricyclo[5.2.1.0^{2,6}]dec-9-yl acrylate (belonging to compounds represented by Formula (1a-1)) and 3,4-epoxytricyclo[5.2.1.0^{2,6}]dec-8-yl acrylate (belonging to compounds represented by Formula (1a-2)) in 170 parts by weight of 3-methoxybutyl acetate. The solution was added dropwise into the flask using a dropping pump over about three hours. Separately, 50 parts by weight of 2,2'-azobis(2,4-dimethylvaleronitrile) as a polymerization initiator was dissolved in 220 parts by weight of 3-methoxybutyl acetate to yield a solution, and this solution was added dropwise into the flask using another dropping pump over about five hours. After the completion of dropwise addition of the polymerization initiator, the mixture was held at the same temperature for about three hours, then cooled to room temperature, and thereby yielded a copolymer solution having a viscosity at 23°C of 100 mPa.s, a solid content of 31.7 percent by weight, and an acid value as a solution of 36.6 mg-KOH/g. The formed copolymer had a weight-average molecular weight (Mw) of 14900 and a molecular weight distribution of 1.67.

### Example 7

The inside of a 1-liter flask equipped with a reflux condenser, dropping funnels, and a stirrer was allowed to be a nitrogen atmosphere by feeding a suitable amount of nitrogen thereto; 160 parts by weight of 3-methoxy-1-butanol and 110 parts by weight of 3-methoxybutyl acetate were placed in the flask and heated to 65°C with stirring. Next, a solution was prepared by dissolving 50 parts by weight of methacrylic acid (MAA), 180 parts by weight of a 50:50 (by mole) mixture (E-DCPA) of 3,4-epoxytricyclo[5.2.1.0^{2,6}]dec-9-yl acrylate (belonging to compounds represented by Formula (1a-1)) and 3,4-epoxytricyclo[5.2.1.0^{2,6}]dec-8-yl acrylate (belonging to compounds represented by Formula (1a-2)), and 70 parts by weight of styrene (ST) in 170 parts by weight of 3-methoxy-1-butanol. The solution was added dropwise into the flask using a dropping pump over about four hours. Separately, 30 parts by weight of 2,2'-azobis(2,4-dimethylvaleronitrile) as a polymerization initiator was dissolved in 225 parts by weight of 3-methoxybutyl acetate to yield a solution, and this solution was added dropwise into the flask using another dropping pump over about five hours. After the completion of dropwise addition of the polymerization initiator, the mixture was held at the same temperature for about four hours, then cooled to room temperature, and thereby yielded a copolymer solution having a viscosity at 23°C of 50 mPa.s, a solid content of 31.6 percent by weight, and an acid value as a solution of 34.3 mg-KOH/g. The formed copolymer had a weight-average molecular weight (Mw) of 7230 and a molecular weight distribution of 1.63.

### Example 8

A copolymer solution was prepared by the procedure of Example 5, except for using, as monomers, 60 parts by weight of methacrylic acid (MAA), 90 parts by weight of a 50:50 (by mole) mixture (E-DCPA) of 3,4-epoxytricyclo[5.2.1.0^{2,6}]dec-9-yl acrylate (belonging to compounds represented by Formula (1a-1)) and 3,4-epoxytricyclo[5.2.1.0^{2,6}]dec-8-yl acrylate (belonging to compounds represented by Formula (1a-2)), 90 parts by weight of 3,4-epoxycyclohexylmethyl methacrylate (E-CHM), and 60 parts by weight of N-cyclohexylmaleimide (CHMI). The copolymer solution had a viscosity at 23°C of 114 mPa.s, a solid content of 32.6 percent by weight, and an acid value as a solution of 34.3 mg-KOH/g. The formed copolymer had a weight-average molecular weight (Mw) of 14700 and a molecular weight distribution of 2.68.

### Example 9

A copolymer solution was prepared by the procedure of Example 5, except for using, as monomers, 55 parts by weight of methacrylic acid (MAA), 45 parts by weight of a 50:50 (by mole) mixture (E-DCPA) of 3,4-epoxytricyclo[5.2.1.0^{2,6}]dec-9-yl acrylate (belonging to compounds represented by Formula (1a-1)) and 3,4-epoxytricyclo[5.2 .1.0^{2,6} ]dec-8-yl acrylate (belonging to compounds represented by Formula (1a-2)), 80 parts by weight of 3,4-epoxycyclohexylmethyl methacrylate (E-CHM), 70 parts by weight of N-cyclohexylmaleimide (CHMI), and 50 parts by weight of methyl methacrylate (MMA). The copolymer solution had a viscosity at 23°C of 110 mPa.s, a solid content of 32.2 percent by weight, and an acid value as a solution of 33.3 mg-KOH/g. The formed copolymer had a weight-average molecular weight (Mw) of 12800 and a molecular weight distribution of 2.38.

### Example 10

A copolymer solution was prepared by the procedure of Example 5, except for using, as monomers, 50 parts by weight of methacrylic acid (MAA), 150 parts by weight of a 50:50 (by mole) mixture (E-DCPA) of 3,4-epoxytricyclo[5.2.1.0^{2,6}]dec-9-yl acrylate (belonging to compounds represented by Formula (1a-1)) and 3,4-epoxytricyclo[5.2.1.0^{2,6}]dec-8-yl acrylate (belonging to compounds represented by Formula (1a-2)), 50 parts by weight of glycidyl methacrylate (GMA), and 50 parts by weight of methyl methacrylate (MMA). The copolymer solution had a viscosity at 23°C of 105 mPa.s, a solid content of 31.5 percent by weight, and an acid value as a solution of 34.5 mg-KOH/g. The formed copolymer had a weight-average molecular weight (Mw) of 9450 and a molecular weight distribution of 2.29.

### Example 11

A copolymer solution was prepared by the procedure of Example 5, except for using, as monomers, 50 parts by weight of methacrylic acid (MAA), 180 parts by weight of a 50:50 (by mole) mixture (E-DCPEA) of 2-(3,4-epoxytricyclo[5.2.1.0^{2,6}]dec-9-yloxy)ethyl acrylate (belonging to compounds represented by Formula (1a-1)) and 2-(3,4-epoxytricyclo[5.2.1.0^{2,6}]dec-8-yloxy)ethyl acrylate (belonging to compounds represented by Formula (1a-2)), and 70 parts by weight of N-cyclohexylmaleimide (CHMI). The copolymer solution had a viscosity at 23°C of 115 mPa.s, a solid content of 31.3 percent by weight, and an acid value as a solution of 34.7 mg-KOH/g. The formed copolymer had a weight-average molecular weight (Mw) of 12500 and a molecular weight distribution of 2.30.

### Example 12

A copolymer solution was prepared by the procedure of Example 5, except for using, as monomers, 50 parts by weight of methacrylic acid (MAA), 180 parts by weight of a 50:50 (by mole) mixture (E-DCPA) of 3,4-epoxytricyclo[5.2.1.0^{2,6}]dec-9-yl acrylate (belonging to compounds represented by Formula (1a-1)) and 3,4-epoxytricyclo[5.2.1.0^{2,6}]dec-8-yl acrylate (belonging to compounds represented by Formula (1a-2)), and 70 parts by weight of dicyclopentyloxyethyl acrylate [i.e., 2-(tricyclo[5.2.1.0^{2,6}]decyloxy)ethyl acrylate] (DCPEA). The polymer solution had a viscosity at 23°C of 118 mPa.s, a solid content of 32.3 percent by weight, and an acid value as a solution of 32.4 mg-KOH/g. The formed copolymer had a weight-average molecular weight (Mw) of 12900 and a molecular weight distribution of 2.29.

### Example 13

The inside of a 1-liter flask equipped with a reflux condenser, dropping funnels, and a stirrer was allowed to be a nitrogen atmosphere by feeding a suitable amount of nitrogen thereto; 200 parts by weight of 3-methoxy-1-butanol and 105 parts by weight of 3-methoxybutyl acetate were placed in the flask and heated to 90°C with stirring. Next, a solution was prepared by dissolving 55 parts by weight of methacrylic acid (MAA), 105 parts by weight of a 50:50 (by mole) mixture (E-DCPA) of 3,4-epoxytricyclo[5.2.1.0^{2,6}]dec-9-yl acrylate (belonging to compounds represented by Formula (1a-1)) and 3,4-epoxytricyclo[5.2.1.0^{2,6}]dec-8-yl acrylate (belonging to compounds represented by Formula (1a-2)), 40 parts by weight of (3-ethyl-3-oxetanyl)methyl methacrylate (OXMA), and 100 parts by weight of N-cyclohexylmaleimide (CHMI) in 140 parts by weight of 3-methoxybutyl acetate. The solution was added dropwise into the flask using a dropping pump over about five hours. Separately, 30 parts by weight of 2,2'-azobis(2,4-dimethylvaleronitrile) as a polymerization initiator was dissolved in 225 parts by weight of 3-methoxybutyl acetate to yield a solution, and this solution was added dropwise into the flask using another dropping pump over about five hours. After the completion of dropwise addition of the polymerization initiator, the mixture was held at the same temperature for about four hours, then cooled to room temperature, and thereby yielded a copolymer solution having a viscosity at 23°C of 88 mPa.s, a solid content of 30.1 percent by weight, and an acid value as a solution of 35.7 mg-KOH/g. The formed copolymer had a weight-average molecular weight (Mw) of 11900 and a molecular weight distribution of 2.08.

### Example 14

A copolymer solution was prepared by the procedure of Example 13, except for using, as monomers, 50 parts by weight of methacrylic acid (MAA), 80 parts by weight of a 50:50 (by mole) mixture (E-DCPEA) of 2-(3,4-epoxytricyclo[5.2.1.0^{2,6}]dec-9-yloxy)ethyl acrylate (belonging to compounds represented by Formula (1a-1)) and 2-(3,4-epoxytricyclo[5.2.1.0^{2,6}]dec-8-yloxy)ethyl acrylate (belonging to compounds represented by Formula (1a-2)), 100 parts by weight of tetrahydrofurfuryl methacrylate (THFMA), and 70 parts by weight of N-cyclohexylmaleimide (CHMI). The copolymer solution had a viscosity at 23°C of 93 mPa.s, a solid content of 31.3 percent by weight, and an acid value as a solution of 32.5 mg-KOH/g. The formed copolymer had a weight-average molecular weight (Mw) of 12530 and a molecular weight distribution of 2.51.

### Example 15

The inside of a 1-liter flask equipped with a reflux condenser, dropping funnels, and a stirrer was allowed to be a nitrogen atmosphere by feeding a suitable amount of nitrogen thereto; 150 parts by weight of 3-methoxy-1-butanol and 110 parts by weight of 3-methoxybutyl acetate were placed in the flask and heated to 90°C with stirring. Next, a solution was prepared by dissolving 60 parts by weight of methacrylic acid (MAA), 160 parts by weight of a 50:50 (by mole) mixture (E-DCPA) of 3,4-epoxytricyclo[5.2.1.0^{2,6}]dec-9-yl acrylate (belonging to compounds represented by Formula (1a-1)) and 3,4-epoxytricyclo[5.2.1.0^{2,6}]dec-8-yl acrylate (belonging to compounds represented by Formula (1a-2)), and 80 parts by weight of (3-ethyl-3-oxetanyl)methyl methacrylate (OXMA) in 170 parts by weight of 3-methoxybutyl acetate. The solution was added dropwise into the flask using a dropping pump over about three hours. Separately, 50 parts by weight of 2,2'-azobis(2,4-dimethylvaleronitrile) as a polymerization initiator was dissolved in 220 parts by weight of 3-methoxybutyl acetate to yield a solution, and this solution was added dropwise into the flask using another dropping pump over about five hours. After the completion of dropwise addition of the polymerization initiator, the mixture was held at the same temperature for about three hours, then cooled to room temperature, and thereby yielded a copolymer solution having a viscosity at 23°C of 98 mPa.s, a solid content of 31.5 percent by weight, and an acid value as a solution of 36.3 mg-KOH/g. The formed copolymer had a weight-average molecular weight (Mw) of 8700 and a molecular weight distribution of 2.06.

### Comparative Example 1

A copolymer solution was prepared by the procedure of Example 5, except for using, as monomers, 55 parts by weight of methacrylic acid (MAA), 175 parts by weight of 3,4-epoxycyclohexylmethyl methacrylate (E-CHM), and 70 parts by weight of N-cyclohexylmaleimide (CHMI). The copolymer solution had a viscosity at 23°C of 125 mPa.s, a solid content of 32.3 percent by weight, and an acid value as a solution of 34.0 mg-KOH/g. The formed copolymer had a weight-average molecular weight (Mw) of 14700 and a molecular weight distribution of 2.47.

### Comparative Example 2

A copolymer solution was prepared by the procedure of Example 5, except for using, as monomers, 60 parts by weight of methacrylic acid (MAA), 50 parts by weight of a 50:50 (by mole) mixture (E-DCPA) of 3,4-epoxytricyclo[5.2.1.0^{2,6}]dec-9-yl acrylate (belonging to compounds represented by Formula (1a-1)) and 3,4-epoxytricyclo[5.2.1.0^{2,6}]dec-8-yl acrylate (belonging to compounds represented by Formula (1a-2)), 130 parts by weight of 3,4-epoxycyclohexylmethyl methacrylate (E-CHM), and 60 parts by weight of N-cyclohexylmaleimide (CHMI). The copolymer solution had a viscosity at 23°C of 115 mPa.s, a solid content of 31.9 percent by weight, and an acid value as a solution of 35.8 mg-KOH/g. The formed copolymer had a weight-average molecular weight (Mw) of 11800 and a molecular weight distribution of 2.15.

### Comparative Example 3

A copolymer solution was prepared by the procedure of Example 5, except for using, as monomers, 50 parts by weight of methacrylic acid (MAA), 60 parts by weight of a 50:50 (by mole) mixture (E-DCPA) of 3,4-epoxytricyclo[5.2.1.0^{2,6}]dec-9-yl acrylate (belonging to compounds represented by Formula (1a-1)) and 3,4-epoxytricyclo[5.2.1.0^{2,6}]dec-8-yl acrylate (belonging to compounds represented by Formula (1a-2)), 50 parts by weight of 3,4-epoxycyclohexylmethyl methacrylate (E-CHM), 70 parts by weight of N-cyclohexylmaleimide (CHMI), and 70 parts by weight of methyl methacrylate (MMA). The copolymer solution had a viscosity at 23°C of 103 mPa.s, a solid content of 32.9 percent by weight, and an acid value as a solution of 34.3 mg-KOH/g. The formed copolymer had a weight-average molecular weight (Mw) of 13700 and a molecular weight distribution of 2.49.

### Evaluation Test

The copolymer solutions prepared according to Examples 5 to 15 and Comparative Examples 1 to 3 were subjected to following evaluation tests. The results are shown in Table 1. In Table 1, numerals in "Monomer composition" represent amounts in terms of part by weight; numerals in "E-DCP (E)A/epoxy" represent the ratios (percent by weight) of the amount of E-DCPA or E-DCPEA to the total amount of epoxy-containing polymerizable unsaturated compounds; and numerals in "Epoxy/copolymer" represent the ratios (percent by weight) of the total amount of epoxy-containing polymerizable unsaturated compounds to the total amount of monomers.

### (1) Storage stability

A radiation-sensitive resin composition as a solution was prepared by mixing 80 parts by weight of each of the copolymer solutions each having a solid content of about 30 percent by weight, prepared according to Examples 5 to 15 and Comparative Examples 1 to 3, 0.5 part by weight of a cationic polymerization initiator (a product of Sanshin Chemical Industry, Co., Ltd., under the trade name of "San-Aid SI-150"), and 40 parts by weight of propylene glycol monomethyl ether acetate, dissolving them with stirring in a mixer for five minutes, and evacuating the solution under reduced pressure. The radiation-sensitive resin composition as a solution was subjected to measurement of viscosity at 23°C, stored at room temperature for one month, and subjected again to measurement of viscosity at 23°C. A sample having an increase in viscosity after storage of less than 30% was evaluated as "Good", and a sample having an increase of 30% or more was evaluated as "Failure" in storage stability.

### (2) Developability

Each of the copolymer solutions prepared according to Examples and Comparative Examples was diluted with a solvent the same as the reaction solvent to a solid content of 3.6 percent by weight. The solution was applied, using a bar coater, to a substrate (stainless steel specified as SUS304 in Japanese Industrial Standards (JIS), 0.5 mm by 80 mm by 80 mm, puffing-finished, one-side Surface Protection Vinyl (SPV)-coated, Nippon Testpanel Co., Ltd., standard test plate), dried in an oven at 120°C for two hours, immersed in an alkali developer (2.35 percent by weight aqueous solution of tetramethylammonium hydroxide) placed in a stainless steel vat about 1 cm deep, and the time for a resin layer to be completely dissolved and removed was measured. A sample having a time to be completely dissolved of three minutes or less was evaluated as "Good", a sample having a time of more than three minutes and less than ten minutes was evaluated as "Fair", and a sample having a time of ten minutes or more was evaluated as "Failure" in developability.

### (3) Optical transparency

A radiation-sensitive resin composition as a solution was prepared by the procedure of Storage Stability Test (1). The solution was filtered through a 0.2-µm Teflon (registered trademark) filter, applied to a glass substrate #1737 (a product of Corning Inc., having a thickness of 0.7 mm and a diameter of 150 mm) to a thickness of 3 µm using a spinner, dried on a hot plate at 90°C for three minutes, and entire surface of which was exposed to light using a highpressure mercury lamp. Next, the entire surface of the coated film was exposed to light using an ultra-highpressure mercury lamp without the interposition of a positive mask pattern, and dried with heating in a clean oven at 120°C for thirty minutes. The minimum transmittance at wavelengths of 400 nm to 800 nm of the substrate bearing a cured film was determined using an ultraviolet spectrophotometer (a product of Hitachi, Ltd., under the trade name of "U-3300"). A sample having a minimum transmittance of 95% or more was evaluated as "Good", a sample having a minimum transmittance of 85% or more and less than 95% was evaluated as "Fair", and a sample having a minimum transmittance of less than 85% was evaluated as "Failure" in optical transparency.

### (4) Thermal stability

A radiation-sensitive resin composition as a solution was prepared by the procedure of Storage Stability Test (1). The solution was filtered through a 0.2-µm Teflon (registered trademark) filter, applied to a glass substrate #1737 (a product of Corning Inc., having a thickness of 0.7 mm and a diameter of 150 mm) to a thickness of 3 µm using a spinner, dried on a hot plate at 90°C for three minutes, and entire surface of which was exposed to light using a highpressure mercury lamp. Next, the coated film was cured by heating in a clean oven at 200°C for thirty minutes, heated again at 230°C for one hour, and the thickness of which was measured. The reduction in thickness between the thickness after curing with heating at 200°C for thirty minutes and the thickness after reheating was determined by calculation. A sample having a reduction in thickness of less than 3% was evaluated as "Good", and a sample having a reduction in thickness of 3% or more was evaluated as "Failure".

[Table 1]

**(Table 1)**

| Monomer composition | Examples | | | | | | | | | | | Comparative Examples | | |
|---|---|---|---|---|---|---|---|---|---|---|---|---|---|---|
| | 5 | 6 | 7 | 8 | 9 | 10 | 11 | 12 | 13 | 14 | 15 | 1 | 2 | 3 |
| E-DCPA | 175 | 240 | 180 | 90 | 45 | 150 | | 180 | 105 | | 160 | | 50 | 60 |
| E-DCPEA | | | | | | | 180 | | | 80 | | | | |
| E-CHM | | | | 90 | 80 | | | | | | | 175 | 130 | 50 |
| GMA | | | | | | 50 | | | | | | | | |
| CHMI | 70 | | | 60 | 70 | | 70 | | 100 | 70 | | 70 | 60 | 70 |
| DCPEA | | | | | | | | 70 | | | | | | |
| OXMA | | | | | | | | | 40 | | 80 | | | |
| THFMA | | | | | | | | | | 100 | | | | |
| ST | | | 70 | | | | | | | | | | | |
| MMA | | | | | 50 | 50 | | | | | | | | 70 |
| MAA | 50 | 60 | 50 | 60 | 55 | 50 | 50 | 50 | 55 | 50 | 60 | 55 | 60 | 50 |
| E-DCP(E)A/ epoxy | 100 | 100 | 100 | 50.0 | 36.0 | 75.0 | 100 | 100 | 100 | 100 | 100 | 0 | 27.8 | 54.5 |
| Epoxy/ copolymer | 58.3 | 80.0 | 60.0 | 60.0 | 41.7 | 66.7 | 60.0 | 60.0 | 40.5 | 40.0 | 72.7 | 58.3 | 60.0 | 36.7 |
| Storage stability | Good | Good | Good | Good | Good | Good | Good | Good | Good | Good | Good | Failure | Failure | Failure |
| Optical transparency | Good | Good | Good | Good | Good | Good | Good | Good | Good | Good | Good | Good | Good | Good |
| Developability | Good | Good | Good | Good | Good | Good | Good | Good | Good | Good | Good | Good | Good | Good |
| Thermal stability | Good | Good | Good | Good | Good | Good | Good | Good | Good | Good | Good | Good | Failure | Failure |

Table 1 demonstrates that the copolymers prepared according to Examples 5 to 15 exhibit superior storage stability and thermal stability while maintaining satisfactory optical transparency and developability, as compared with the copolymers prepared according to Comparative Examples 1 to 3.

### Industrial Applicability

Reaction mixtures (reaction solutions) containing unsaturated carboxylic acid esters containing an 3,4-epoxytricyclo[5.2.1.0^{2,6}]decane skeleton, represented by Formula (1a) (cycloaliphatic epoxy (meth)acrylate) prepared by a process according to the present invention can be used as reactive solvents without further treatment. In other words, they can be used as reactive diluents that can be cured by the action of active energy rays such as ultraviolet rays. The solutions can also be used as additives for various polymers and other compounds after separation or as intact without separation. The compounds can be reacted upon epoxy group with a halogen to convert into halogenated alicyclic group-containing unsaturated carboxylic acid esters and used as flame-retardant radical polymerizable monomers. Alternatively, the compounds can be reacted with other compounds to yield intermediates for the synthetic preparation of materials for the production of curable coatings, inks, adhesives, sealants, and molded articles. The compounds, for example, can be used as acrylic resins having a cycloaliphatic epoxy group in a side chain by copolymerizing with a radical polymerizable monomer. They can also be used as curable acrylic resins having an unsaturated group with or without a cycloaliphatic epoxy group in a side chain by reacting all or part of cycloaliphatic epoxy groups in side chain with a carboxylic acid having an unsaturated group, such as acrylic acid.
Such curable acrylic resins can be used, for example, as active energy ray-curable resins for photoresists. The compounds can also be used, as curable monomers, for acid removers, furniture coating agents, ornament coating agents, automobile under coaters, sealers, finishing coating agents, coating agents for beverage cans and other cans, inks for alphabetic information or image information, or sealants for electronic parts. Unsaturated carboxylic acid esters having an 3,4-epoxytricyclo[5.2.1.0^{2,6}]decane skeleton (cycloaliphatic epoxy (meth)acrylate) according to the present invention can be converted into resins for photoresists or for casting printing roll suitable for the development of a printing matrix or a printed circuit board, by impregnating a resin with the compounds by the action of the reactivity of epoxy group of the compounds. In addition, they can be converted into compositions for molding mainly containing an unsaturated polyester and styrene and being reinforced with glass, carbon, graphite or another fiber. The compositions can be combined with a sheet-forming component and molded into molded articles.

Copolymers according to the present invention are suitably used as radiation-sensitive resins such as resins for photoresists using g-ray, i-ray, or excimer laser such as XeCl , KrF, KrCl , ArF, ArCl , F₂, Kr₂, KrAr, or Ar₂ laser. They are typically suitably used as resins for photoresists soluble in an aqueous alkali solution (resins for negative-working photoresists).

## Claims

1. An unsaturated carboxylic acid ester containing an 3,4-epoxytricyclo[5.2.1.0^{2,6}]decane skeleton, represented by following Formula (1a): 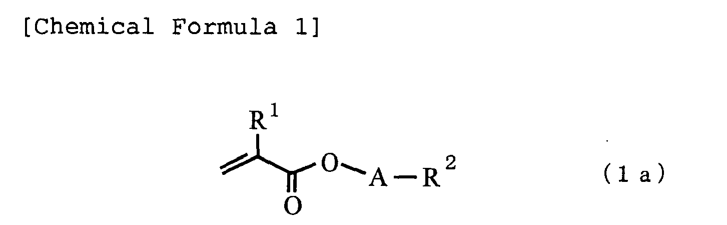 wherein R¹ represents a hydrogen atom or an alkyl group having one to seven carbon atoms which may be substituted with hydroxyl group; "A" represents a single bond or a bivalent hydrocarbon group which may contain hetero atom; and R² represents a group having an 3,4-epoxytricyclo[5.2.1.0^{2,6}]decane skeleton, represented by following Formula (1b): 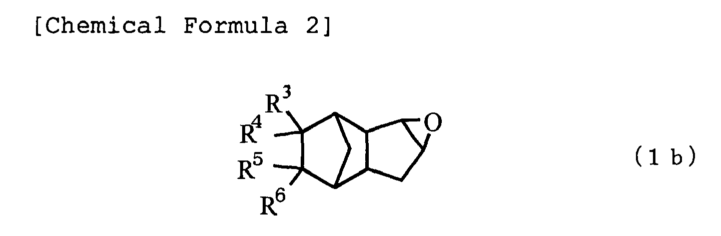 wherein one of R³, R⁴, R⁵, and R⁶ represents a bond with "A" in Formula (1a) and the others each represent a hydrogen atom or an alkyl group having one to ten carbon atoms.

2. The unsaturated carboxylic acid ester containing an 3,4-epoxytricyclo[5.2.1.0^{2,6}]decane skeleton according to claim 1, wherein "A" in Formula (1a) is a group represented by following Formula (2): 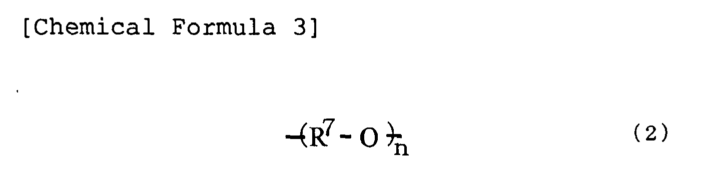 wherein R⁷ represents a bivalent hydrocarbon group having one to twelve carbon atoms; and "n" represents an integer of 0 or more.

3. The unsaturated carboxylic acid ester containing an 3,4-epoxytricyclo[5.2.1.0^{2,6}]decane skeleton according to claim 2, wherein R⁷ is an ethylene group; and "n" is an integer of 0 to 5.

4. A process for the preparation of an unsaturated carboxylic acid ester containing an 3,4-epoxytricyclo[5.2.1.0^{2,6}]decane skeleton, comprising the step of carrying out epoxidation of an unsaturated carboxylic acid ester containing a tricyclo[5.2 .1.0^{2,6}]dec-3-ene skeleton, represented by following Formula (3a): 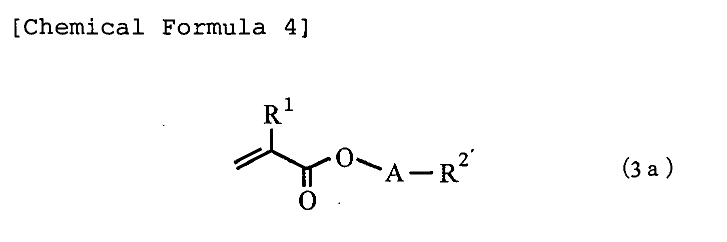 wherein R¹ represents a hydrogen atom or an alkyl group having one to seven carbon atoms which may be substituted with hydroxyl group; "A" represents a single bond or a bivalent hydrocarbon group which may contain hetero atom; and R²' represents a group having a tricyclo[5.2.1.0^{2,6}] dec-3-ene skeleton, represented by following Formula (3b): 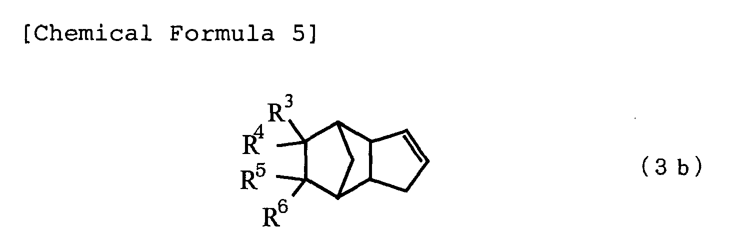 wherein one of R³, R⁴, R⁵, and R⁶ represents a bond with "A" in Formula (3a), and the others each represent a hydrogen atom or an alkyl group having one to ten carbon atoms, in the presence of an epoxidizing agent selected from the group consisting of a peracid prepared as a result of oxidation of an aldehyde; hydrogen peroxide; and a peracid derived from hydrogen peroxide and an organic acid, to yield an unsaturated carboxylic acid ester containing an 3,4-epoxytricyclo[5.2,1.0^{2,6}] decane skeleton, represented by following Formula (1a): 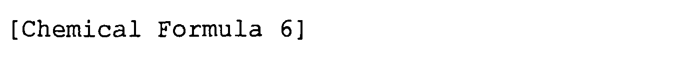 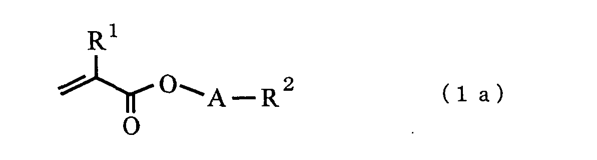 wherein R¹ represents a hydrogen atom or an alkyl group having one to seven carbon atoms which may be substituted with hydroxyl group; "A" represents a single bond or a bivalent hydrocarbon group which may contain hetero atom; and R² represents a group having an 3,4-epoxytricyclo[5.2.1.0^{2,6}]decane skeleton, represented by following Formula (1b): 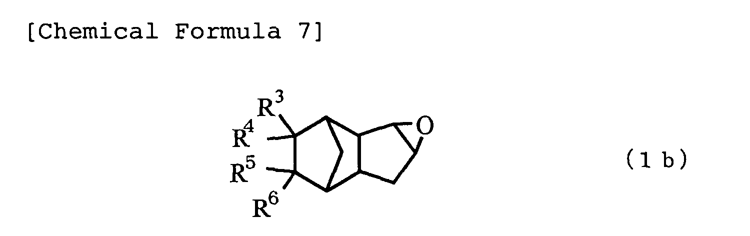 wherein one of R³, R⁴, R⁵, and R⁶ represents a bond with "A" in Formula (1a), and the others each represent a hydrogen atom or an alkyl group having one to ten carbon atoms.

5. The process for the preparation of an unsaturated carboxylic acid ester containing an 3,4-epoxytricyclo[5.2.1.0^{2,6}]decane skeleton according to claim 4, wherein the peracid is peracetic acid.

6. The process for the preparation of an unsaturated carboxylic acid ester containing an 3,4-epoxytricyclo[5.2.1.0^{2,6}]decane skeleton according to claim 4, wherein the epoxidation is carried out in the presence of hydrogen peroxide as the epoxidizing agent and further in the presence of a phase transfer catalyst and a metal salt of tungstic acid.

7. A copolymer comprising monomeric units corresponding to an unsaturated carboxylic acid ester containing an 3,4-epoxytricyclo[5.2.1.0^{2,6}]decane skeleton, represented by following Formula (1a): 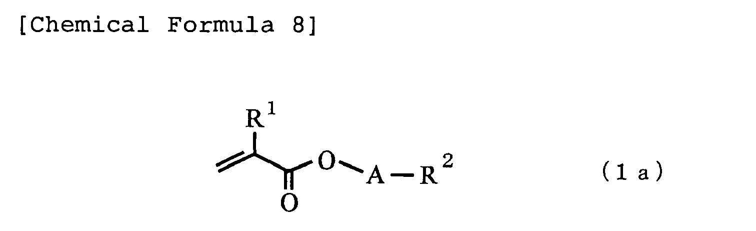 wherein R¹ represents a hydrogen atom or an alkyl group having one to seven carbon atoms which may be substituted with hydroxyl group; "A" represents a single bond or a bivalent hydrocarbon group which may contain hetero atom; and R² represents a group having an 3,4-epoxytricyclo[5.2.1.0^{2,6}]decane skeleton, represented by following Formula (1b): 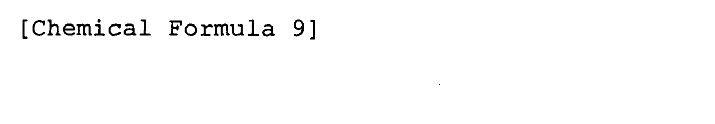 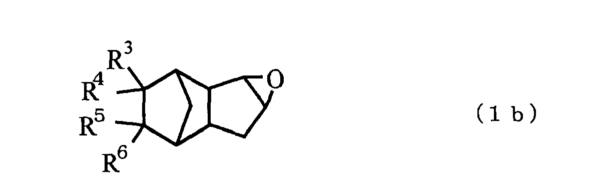 wherein one of R³, R⁴, R⁵, and R⁶ represents a bond with "A" in Formula (1a), and the others each represent a hydrogen atom or an alkyl group having one to ten carbon atoms, wherein the copolymer is a copolymer including monomeric units (A) containing an alkali-soluble group and monomeric units (B) corresponding to an epoxy-containing polymerizable unsaturated compound, or a copolymer including, in addition to the monomeric units (A) and (B), monomeric units (C) corresponding to at least one epoxy-free polymerizable unsaturated compound selected from the group consisting of (c1) an alkyl-substituted or -unsubstituted styrene, (c2) an unsaturated carboxylic acid ester represented by following Formula (4): 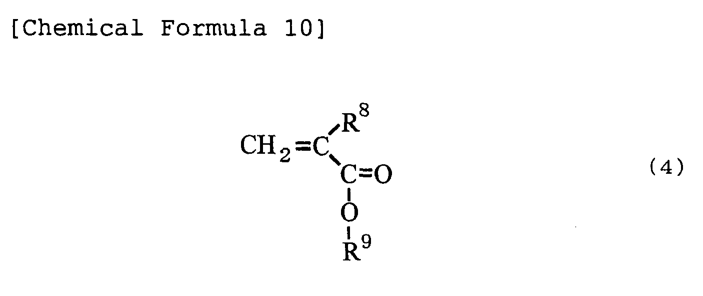 wherein R⁸ represents a hydrogen atom or an alkyl group having one to seven carbon atoms; R⁹ represents a primary or secondary alkyl group having one to twelve carbon atoms, an alkenyl group having two to twelve carbon atoms, an aryl group, an aralkyl group, a group containing a cyclic ether having four or more members, or a-(R¹⁰-O)ₘ-R¹¹ group, wherein R¹⁰ represents a bivalent hydrocarbon group having one to twelve carbon atoms; R¹¹ represents a hydrogen atom or a hydrocarbon group; and "m" represents an integer of 1 or more,
and (c3) an N-substituted maleimide, wherein the monomeric units (B) occupy 40 to 90 percent by weight of total monomeric units, and wherein 30 percent by weight or more of the monomeric units (B) are each a monomeric unit corresponding to the unsaturated carboxylic acid ester containing an 3,4-epoxytricyclo[5.2.1.0^{2,6}]decane skeleton, represented by Formula (1a).

8. The copolymer according to claim 7, wherein the monomeric units (A) occupy 10 to 50 percent by weight of total monomeric units.

9. The copolymer according to one of claims 7 and 8, as a resin for a photoresist soluble in an aqueous alkali solution.

10. A process for the preparation of a copolymer, comprising the step of subjecting a monomer mixture to copolymerization, wherein the monomer mixture is a monomer mixture containing a polymerizable unsaturated compound (a) having an alkali-soluble group, and an epoxy-containing polymerizable unsaturated compound (b), or is a monomer mixture containing, in addition to the compounds (a) and (b), at least one epoxy-free polymerizable unsaturated compound (c) selected from the group consisting of (c1) an alkyl-substituted or -unsubstituted styrene, (c2) an unsaturated carboxylic acid ester represented by following Formula (4): 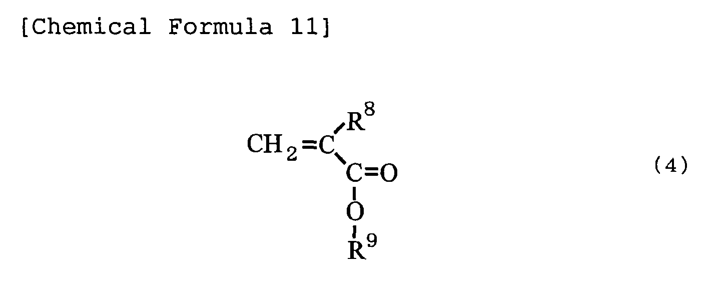 wherein R⁸ represents a hydrogen atom or an alkyl group having one to seven carbon atoms; R⁹ represents a primary or secondary alkyl group having one to twelve carbon atoms, an alkenyl group having two to twelve carbon atoms, an aryl group, an aralkyl group, a group containing a cyclic ether having four or more members, or a-(R¹⁰-O)ₘ-R ¹¹ group, wherein R¹⁰ represents a bivalent hydrocarbon group having one to twelve carbon atoms; R¹¹ represents a hydrogen atom or a hydrocarbon group; and "m" represents an integer of 1 or more, and (c3) an N-substituted maleimide, wherein the epoxy-containing polymerizable unsaturated compound (b) occupies 40 to 90 percent by weight of total monomers, and wherein 30 percent by weight or more of the epoxy-containing polymerizable unsaturated compound (b) is an unsaturated carboxylic acid ester containing an 3,4-epoxytricyclo[5.2.1.0^{2,6}]decane skeleton, represented by following Formula (1a): 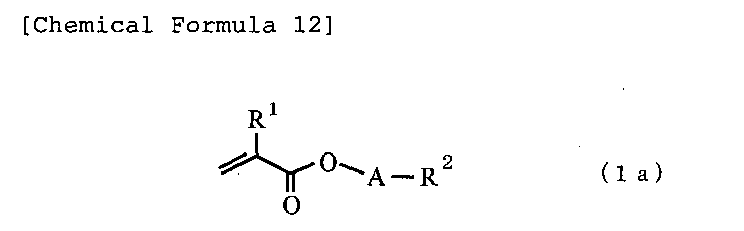 wherein R¹ represents a hydrogen atom or an alkyl group having one to seven carbon atoms which may be substituted with hydroxyl group; "A" represents a single bond or a bivalent hydrocarbon group which may contain hetero atom; and R² represents a group having an 3,4-epoxytricyclo[5.2.1.0^{2,6}]decane skeleton, represented by following Formula (1b): 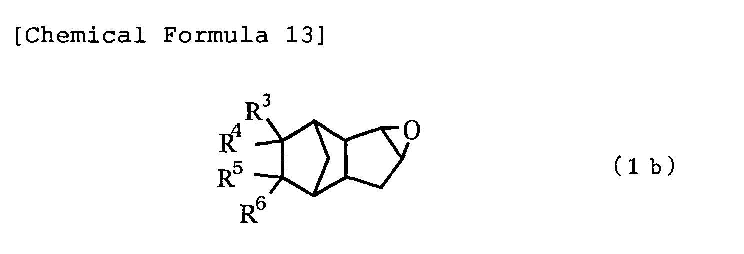 wherein one of R³, R⁴, R⁵, and R⁶ represents a bond with "A" in Formula (1a), and the others each represent a hydrogen atom or an alkyl group having one to ten carbon atoms.
